# EUROPÄISCHE PATENTSCHRIFT

(11) **EP 1 692 115 B1**
(45) Veröffentlichungstag und Bekanntmachung des Hinweises auf die Patenterteilung: **19.12.2007**
(21) Anmeldenummer: 04803387.2
(22) Anmeldetag: 01.12.2004
(51) Int. Cl.: C07D 239/56, C07D 239/54

(54) **VERFAHREN ZUR HERSTELLUNG VON 3-PHENYL(THIO)URACILEN UND 3-PHENYLDITHIOURACILEN**
METHOD FOR PRODUCING 3-PHENYL(THIO)URACILS AND 3-PHENYLDITHIOURACILS
PROCÉDÉ DE PRODUCTION DE 3-PH NYL(THIO)URACILES ET DE 3-PH NYLDITHIOURACILES

(30) Priorität: 03.12.2003 DE 10356474
(43) Veröffentlichungstag der Anmeldung: 23.08.2006
(73) Patentinhaber: BASF Aktiengesellschaft, 67056 Ludwigshafen (DE)
(72) Erfinder: HAMPRECHT, Gerhard, 69469 Weinheim (DE); PUHL, Michael, 68623 Lampertheim (DE); WOLF, Bernd, 67136 Fussgönheim (DE); KEIL, Michael, 67251 Freinsheim (DE); REINHARD, Robert, 67065 Ludwigshafen (DE); SEITZ, Werner, 68723 Plankstadt (DE); MAYER, Guido, 67161 Gönnheim (DE)
(86) Internationale Anmeldenummer: PCT/EP2004/013615
(87) Internationale Veröffentlichungsnummer: WO 2005/054208

(56) Entgegenhaltungen:
- EP-A- 0 545 206
- EP-A- 0 831 091
- WO-A-01/83459
- WO-A1-03/024221
- WO-A1-03/097589
- DE-A1- 19 741 411
- US-A- 5 169 430
- A. M. KAMAL EL-DEAN AND M.E.ABDEL-MONEAM: "synthesis of pyrimidines, thienopyrimidines and pyrazolopyrimidine" J. OF CHINESE CHEM. SOC., Bd. 49, 2002, Seiten 1057-1060, XP009046134

## Beschreibung

Die vorliegende Erfindung betrifft ein Verfahren zur Herstellung von 3-Phenyl(thio)-uracilen und 3-Phenyldithiouracilen der Formel I 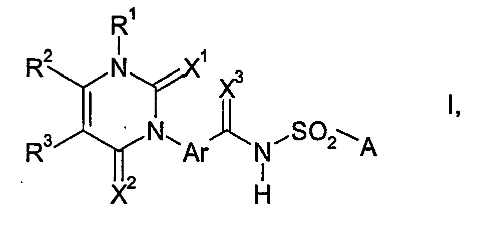 worin die variablen die folgenden Bedeutungen haben:
- R¹: Wasserstoff, Cyano, Amino, C₁-C₆-Alkyl, C₁-C₃-Cyanoalkyl, C₁-C₆-Halogenalkyl, C₁-C₆-Halogenalkoxy, C₃-C₇-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl;
- R² und R³: unabhängig voneinander Wasserstoff, C₁-C₆-Alkyl, C₁-C₆-Halogenalkyl, C₃-C₇-Cycloalkyl, C₂-C₆-Alkenyl, C₂-C₆-Halogenalkenyl, C₃-C₆-Alkinyl oder C₃-C₆-Halogenalkinyl;
- X¹, X² und X³: unabhängig voneinander Sauerstoff oder Schwefel;
- Ar: Phenyl, das durch folgende Gruppen ein- oder mehrfach substituiert sein kann: Wasserstoff, Halogen, Cyano, C₁-C₄-Alkyl oder C₁-C₄-Halogenalkyl; und
- A: ein von einem primären oder sekundären Amin abgeleiteter Rest oder NH₂.

3-Phenyluracile der Formel I und die entsprechenden Thio- und Dithiouracile sind prinzipiell aus der WO 01/83459 bekannt. Ihre Herstellung erfolgt gemäß der in WO 01/83459 angegebenen Lehre durch die folgenden Verfahren A bis C. In den nachfolgenden Schemata A bis C haben die Variablen Ar und A die zuvor genannten Bedeutungen, Hal steht für Halogen und Q steht für einen gegebenenfalls substituierten Uracil-, Thiouracil- oder Dithiouracilrest:
(A) Kondensation einer substituierten Benzoesäure mit einem substituierten Sulfamidsäureamid in Gegenwart von N,N-Carbonyldiimidazol (CDI) oder Umwandlung der Carbonsäure in ihr Säurechlorid und anschließende Umsetzung des Säurechlorids mit dem Sulfamidsäureamid gemäß folgendem Schema A: 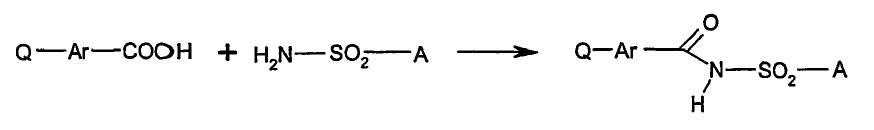
   Nachteilig an dieser Vorgehensweise ist, dass die eingesetzte Benzoesäure erst durch Spaltung mit Bortribromid bei entsprechendem Salzanfall aus dem vorangehenden Ester erhältlich ist. Zudem liegt die Ausbeute der Kondensation mit Sulfamidsäureamiden nur zwischen 16 und 45 %. Auch der Umweg über ein vorher hergestelltes Säurechlorid führt in nur 26 % Ausbeute zu dem gewünschten Benzoylsulfamidsäureamid, das zudem chromatographisch von seinen Verunreinigungen befreit werden muss.
(B) Ersatz eines Halogenatoms durch einen Uracil-, Thiouracil- oder Dithiouracilrest gemäß folgendem Schema B: 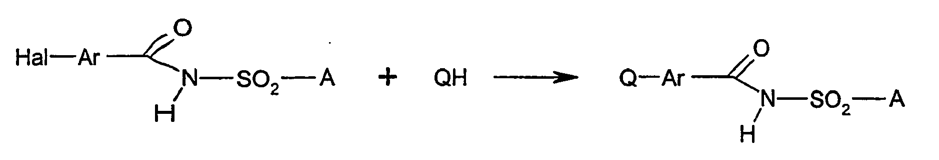
   Das Verfahren B weist den Nachteil auf, dass der eingesetzte Halogenaromat erst umständlich über eine Sandmeyer-Reatkion bereitgestellt werden muss. Außerdem ist die Selektivität der Reaktion bezüglich des Halogenrestes bei Vorliegen weiterer Halogensubstituenten an Ar unbefriedigend.
(C) Umsetzung einer Anilinverbindung mit einem Oxazinon und anschließende Alkylierung des erhaltenen 3-Phenyluracils in Gegenwart einer Base gemäß folgendem Schema C:

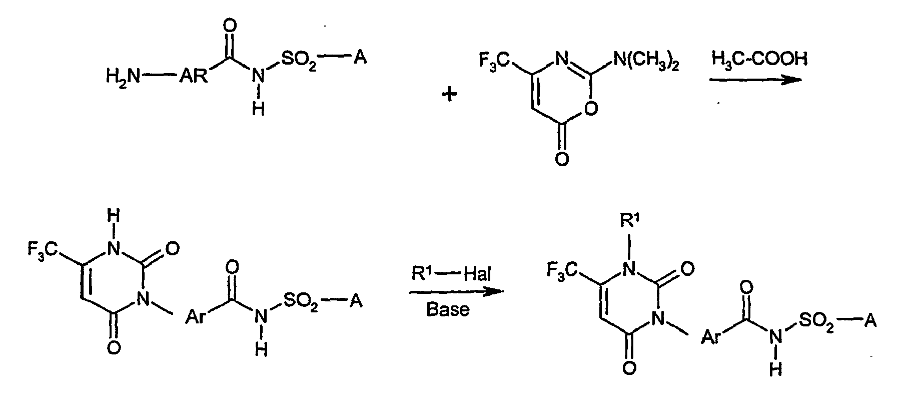

Hierbei hat die variable R¹ die zuvor genannten Bedeutungen.

Von Nachteil ist, dass das verwendete Oxazinon erst aufwendig durch Umsetzung eines Aminocrotonsäureesters mit einem Dialkylcarbamoylchlorid und anschließende Zyklisierung mit Phosphoroxychlorid, Phosphorpentachlorid oder Oxalylchlorid hergestellt werden muss. Dieses Verfahren ist ebenfalls aufgrund der eingesetzten Ausgangsmaterialien und der Reaktionsstufen nicht ausreichend wirtschaftlich.

Es ist bekannt, dass man 3-Phenyluracile durch Umsetzung von Phenylisocyanaten mit Aminoalkencarbonsäureestem herstellen kann, siehe z. B. EP 0 831 091. Allerdings weisen die in der EP 0 831 091 eingesetzten Phenylisocyanate keine Acylsulfonamidgruppe auf.

Andererseits ist bekannt, dass Iso(thio)cyanatgruppen mit Sulfonamid-Gruppen eine Vielzahl verschiedener Reaktionen eingehen können. So können lso(thio)cyanantgruppen mit Sulfonamidgruppen, die am Stickstoffatom ein Wasserstoffatom tragen, unter Bildung von Sulfonylharnstoffen reagieren. So beschreiben beispielsweise J. Cervello und T. Sastre in Synthesis 1990, 221-222, die Umsetzung von Tolylsulfonamiden mit Arylisocyananten, wobei der entsprechende N-Tosylhamstoff gebildet wird.

Aus der US 4,309,209 ist bekannt, dass Phenylisocyanate mit Chlormethan-(N-methyl)sulfonamid (= CICH₂SO₂NHCH₃) unter Bildung eines 1,2,4-Thiadiazolidin-1,1,3-trions reagiert.

P. Schwenkkraus und H.-H. Otto beschreiben in Arch. Pharm. (Weinheim) 326, 437 - 441 (1993) die Umsetzung von 3-Halogenalkyl-β-sultamen , also cyclischen Sulfonamiden, mit Phenylisocyanat unter Bildung von Carbamoylverbindungen.

Aus der DE 3433391 ist die Umsetzung des cyclischen Sulfonamids Saccharin mit Acylisocyanaten zu N-acylierten Saccharin-Derivaten bekannt.

B. A. Arbuzov, N. N. Zobova und N. R. Fedotava beschreiben in JZV Akad Nauk SSSR, Ser Khim 1990, 2874 (engl. Übersetzung: Bulletin of the Academy of Sciences of the USSR, Division of Chemical Sciences, Bd. 39, (1990) S. 2610) die N- und O-Acylierung von Saccharin durch Umsetzung mit einem Trifluoracetylisocyanat.

Der vorliegenden Erfindung liegt die Aufgabe zugrunde, ein einfaches und wirtschaftliches Verfahren zur Herstellung von 3-Phenyl(thio)uracilen und -dithiouracilen bereitzustellen, mit dem hohe Ausbeuten und hohe Reinheit an Wertprodukt erzielt werden.

Es wurde überraschenderweise gefunden, dass diese Aufgabe durch ein Verfahren gelöst wird, bei dem man ein Phenyliso(thio)cyanat der Formel II 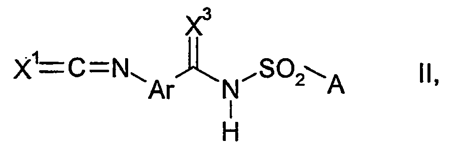 worin die Variablen X¹, X³, Ar und A die zuvor genannten Bedeutungen aufweisen,
mit einem Enamin der Formel III 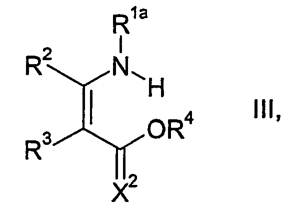 umsetzt, worin
- R^{1a}: die zuvor für R¹ genannten Bedeutungen mit Ausnahme von Amino aufweist;
- R², R³ und X²: die zuvor genannten Bedeutungen aufweisen; und
- R⁴: für C₁-C₆-Alkyl, C₁-C₆-Halogenalkyl, C₁-C₃-Alkoxy-C₁-C₃-alkyl, C₁-C₃-Alkthio-C₁-C₃-alkyl, C₂-C₆-Alkenyl, C₂-C₆-Halogenalkenyl, C₃-C₆-Alkinyl, C₃-C₆-Halogenalkinyl, C₃-C₇-Cycloalkyl, C₁-C₆-Cyanoalkyl oder Benzyl,
das seinerseits unsubstituiert oder am Phenylring durch Methyl, Methoxy, Methylthio, Halogen, Nitro oder Cyano substituiert ist,
vorzugsweise für C₁-C₆-Alkyl, C₁-C₆-Halogenalkyl, C₂-C₆-Alkenyl, C₂-C₆-Halogenalkenyl, C₃-C₆-Alkinyl, C₃-C₆-Halogenalkinyl oder C₁-C₃-Alkoxy-C₁-C₃-alkyl
steht; in Gegenwart von 1,8 bis 2,6 Äquivalenten Basen pro Mol des Phenyliso(thio)cyanates der Formel II;
und gegebenenfalls in einem weiteren Schntt die Umsetzung des erhaltenen 3-Phenyl(thio)uracils oder 3-Phenyldithiouracils der Formel I mit R¹=R^{1a}, wenn R¹ für Wasserstoff steht, mit einem Aminierungsmittel der Formel IV

H₂N-L¹ IV,

wobei L¹ für eine nucleophile Abgangsgruppe steht,
zu 3-Phenyl(thio)uracilen oder 3-Phenyldithiouracilen der Formel I mit R¹=Amino umsetzt.

Demnach betrifft die vorliegende Erfindung ein Verfahren zur Herstellung der 3-Phenyl(thio)uracile oder 3-Phenyldithiouracile der Formel I mit R¹=R^{1a}, das die Umsetzung eines Phenyliso(thio)cyanates der Formel II mit einem Enamin der Formel III umfasst.

Ein weiterer Gegenstand der vorliegenden Erfindung ist ein Verfahren zur Herstellung der vorstehend definierten 3-Phenyl(thio)uracile oder 3-Phenyldithiouracile der Formel I, wobei R¹ nicht für Wasserstoff steht, bei dem man die nach dem erfindungsgemäßen Verfahren erhaltenen Verbindungen I mit R¹ = Wasserstoff mit einem Aminierungsmittel oder Alkylierungsmittel umsetzt.

Nach dem erfindungsgemäßen Verfahren werden 3-Phenyl(thio)uracile und -dithiouracile der Formel I in hohen Ausbeuten und hohen Reinheiten erhalten. Dies überrascht angesichts der Tatsache, dass das eingesetzte Substrat sowohl eine Iso(thio)cyanatgruppe als auch eine Sulfonamidgruppe aufweist, die miteinander reagieren können und somit eine Vielzahl von Nebenreaktionen, einschließlich Oligomer- oder Polymerbildung hätten erwarten lassen.

Die bei der Definition der Substituenten oder als Reste an Phenyl, Naphthyl oder heterocyclischen Ringen genannten organischen Molekülteile stellen - wie die Bedeutung Halogen - Sammelbegriffe für individuelle Aufzählungen der einzelnen Gruppenmitglieder dar, wobei der Ausdruck Cₙ-Cₘ die mögliche Anzahl der Kohlenstoffatome im Molekülteil angibt. Sämtliche Kohlenstoffketten, also alle Alkyl-, Alkenyl- und Alkinylteile können geradkettig oder verzweigt sein. Soweit nicht anders angegeben, tragen halogenierte Substituenten vorzugsweise ein bis sechs gleiche oder verschiedene Halogenatome. Die Bedeutung Halogen steht jeweils für Fluor, Chlor, Brom oder Iod.

Ferner stehen beispielsweise:
- C₁-C₄-Alkyl für: z. B. Methyl, Ethyl, Propyl, 1-Methylethyl, Butyl, 1-Methylpropyl, 2-Methylpropyl oder 1,1-Dimethylethyl;
- C₁-C₁₀-Alkyl: ein gesättigter aliphatischer Kohlenwasserstoffrest mit 1 bis 10 C-Atomen, z. B. C₁-C₄-Alkyl, wie voranstehend genannt, sowie z. B. n-Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl, 1-Ethyl-3-methylpropyl, n-Heptyl, n-Nonyl, n-Decyl, 1-Methylhexyl, 1-Ethylhexyl, 1-Methylheptyl, 1-Methyloctyl, 1-Methylnonyl;
- C₂-C₁₀-Alkenyl: ein einfach ungesättigter olefinischer Kohlenwasserstoffrest mit 2 bis 10 C-Atomen, vorzugsweise 3 bis 6 C-Atomen, z. B. Ethenyl, Prop-2-en-1-yl (= Allyl), Prop-1-en-1-yl, But-1-en-4-yl, But-2-en-1-yl, But-3-en-1-yl, 1-Methylprop-2-en-1-yl, 2-Methylprop-2-en-1-yl, 1-Penten-3-yl, 1-Penten-4-yl, 2-Penten-4-yl, 1-Methylbut-2-en-1-yl, 2-Methylbut-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, 1-Ethylprop-1-en-2-yl, n-Hex-1-en-1-yl, n-Hex-2-en-1-yl, Hex-3-en-1-yl, Hex-4-en-1-yl, Hex-5-en-1-yl, 1-Methyl-pent-1-en-1-yl, 2-Methylpent-1-en-1-yl, 3-Methylpent-1-en-1-yl, 4-Methylpent-1-en-1-yl, 1-Methylpent-2-en-1-yl, 2-Methylpent-2-en-1-yl, 3-Methylpent-2-en-1-yl, 4-Methylpent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethylbut-2-en-1-yl, 1-Ethylbut-3-en-1-yl, 2-Ethylbut-2-en-1-yl, 2-Ethylbut-3-en-1-yl, 1,1,2-Trimethylprop-2-en-1-yl, 1-Ethyl-1-methylprop-2-en-1-yl, 1-Ethyl-2-methylprop-2-en-1-yl, Hept-2-en-1-yl, Oct-2-en-1-yl, Non-2-en-1-yl, Dec-2-en-1-yl;
- C₂-C₁₀-Alkinyl: ein Kohlenwasserstoffrest mit 2 bis 10 C-Atomen, vorzugsweise 3 bis 6 C-Atomen und einer Dreifachbindung, z. B. Ethinyl, Prop-2-in-1-yl (= Propargyl), Prop-1-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in 5-yl, 3-Methylbut-1-in-3-yl, 3-Methylbut-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methylpent-1-in-3-yl, 3-Methylpent-1-in-4-yl, 3-Methylpent-1-in-5-yl, 4-Methylpent-2-in-4-yl, 4-Methylpent-2-in-5-yl, Hept-2-in-1-yl, Oct-2-in-1-yl, Non-2-in-1-yl, Dec-2-in-1-yl;
- C₁-C₄-Halogenalkyl für: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifiluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₁₀-Halogenalkyl: C₁-C₁₀-Alkyl wie vorstehend genannt, worin 1 bis 6 Wasserstoffatome durch Halogenatome, vorzugsweise durch Fluor und/oder Chlor substituiert sind, z. B.: C₁-C₄-Halogenalkyl, wie vorstehend genannt, sowie 5-Fluorpentyl, 5-Chlorpentyl, 5-Brompentyl, 5-lodpentyl, Undecafluorpentyl, 6-Fluorhexyl, 6-Chlorhexyl, 6-Bromhexyl, oder 6-Iodhexyl;
- C₂-C₁₀-Halogenalkenyl: C₂-C₁₀-Alkenyl wie vorstehend genannt, worin 1 bis 6 Wasserstoffatome durch Halogenatome, vorzugsweise durch Fluor und/oder Chlor substituiert sind: z. B. 2-Chlorallyl, 3-Chlorallyl, 2,3-Dichlorallyl, 3,3-Dichlorallyl, 2,3,3-Trichlorallyl, 2,3-Dichlorbut-2-en-1-yl, 2-Bromallyl, 3-Bromallyl, 2,3-Dibromallyl, 3,3-Dibromallyl, 2,3,3-Tribromallyl oder 2,3-Dibrombut-2-en-1-yl;
- C₂-C₁₀-Halogenalkinyl: C₂-C₁₀-Alkinyl wie vorstehend genannt, worin 1 bis 6 Wasserstoffatome durch Halogenatome, vorzugsweise durch Fluor und/oder Chlor substituiert sind: z. B. 1,1-Difluorprop-2-in-1-yl, 1,1-Difluorbut-2-in-1-yl, 4-Fluorbut-2-in-1-yl, 4-Chlorbut-2-in-1-yl, 5-Fluorpent-3-in-1-yl oder 6-Fluorhex-4-in-1-yl;
- C₁-C₁₀-Cyanoalkyl: durch eine CN-Gruppe substituiertes C₁-C₁₀-Alkyl, z. B Cyanomethyl, 1-Cyanoethyl, 2-Cyanoethyl, 1-Cyanopropyl, 2-Cyanopropyl, 3-Cyanopropyl, 1-Cyanoprop-2-yl, 2-Cyanoprop-2-yl, 1-Cyanobutyl, 2-Cyanobutyl, 3-Cyanobutyl, 4-Cyanobutyl, 1-Cyanobut-2-yl, 2-Cyanobut-2-yl, 1-Cyanobut-3-yl, 2-Cyanobut-3-yl, 1-Cyano-2-methylprop-3-yl, 2-Cyano-2-methylprop-3-yl, 3-Cyano-2-methylprop-3-yl, 3-Cyano-2,2-dimethylpropyl, 6-Cyanohex-1-yl, 7-Cyanohept-1-yl, 8-Cyanooct-1-yl, 9-Cyanonon-1-yl, 10-Cyanodec-1-yl;
- C₃-C₁₀-Cycloalkyl: für einen cycloaliphatischen Rest mit 3 bis 10 C-Atomen: z B. Cyclopropyl, Cyclobutyl, Cyclopentyl, Cyclohexyl, Cycloheptyl, Cyclooctyl, Cyclononyl oder Cyclodecyl;
- C₃-C₁₀-Cycloalkenyl: für einen cycloaliphatischen Rest mit 3 bis 10 C-Atomen und einer Doppelbindung: z. B. Cyclopropen-1-yl, Cyclobuten-1-yl, Cyclopenten-1-yl, Cyclohexen-1-yl, Cyclohepten-1-yl, Cycloocten-1-yl, Cyclononen-1-yl, Cyclodecen-1-yl, Cyclopent-2-en-1-yl, Cyclohex-2-en-1-yl, Cyclohept-2-en-1-yl, Cyclooct-2-en-1-yl, Cyclonon-2-en-1-yl, Cyclodec-2-en-1-yl, Cyclohex-3-en-1-yl, Cyclohept-3-en-1-yl, Cyclooct-3-en-1-yl, Cyclooct-4-en-1-yl, Cyclonon-3-en-1-yl, Cyclonon-4-en-1-yl, Cyclodec-4-en-1-yl oder Cyclodec-3-en-1-yl;
- C₁-C₄-Alkylcarbonyl: für einen über eine Carbonylgruppe gebundenen Alkylrest mit 1 bis 4 C-Atomen, z. B. für Acetyl, Propionyl, Butyryl oder Isobutyryl;
- (C₁-C₄-Alkylamino)carbonyl: z. B. Methylaminocarbonyl, Ethylaminocarbonyl, Propylaminocarbonyl, 1-Methylethylaminocarbonyl, Butylaminocarbonyl, Methylpropylaminocarbonyl, 2-Methylpropylaminocarbonyl oder 1,1-Dimethylethylaminocarbonyl;
- Di-(C₁-C₄-alkyl)-aminocarbonyl: z. B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropylpaminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl N-ethylaminocarbonyl, N-Ethyl-N-(1-methylpropylraminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- C₁-C₄-Alkoxy: für einen über ein Sauerstoffatom gebundenen Alkylrest mit 1 bis 4 C-Atomen, z. B. Methoxy, Ethoxy, Propoxy, 1-Methylethoxy, Butoxy, 1-Methylpropoxy, 2-Methylpropoxy oder 1,1-Dimethylethoxy;
- C₁-C₄-Alkoxycarbonyl: für einen über eine Carbonylgruppe gebundenen Alkoxyrest mit 1 bis 4 C-Atomen, z. B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₄-Alkylthio (C₁-C₄-Alkylsulfanyl: C₁-C₄-Alkyl-S-): für einen über ein Schwefelatom gebundenen Alkylrest mit 1 bis 4 C-Atomen, z.B. Methylthio, Ethylthio, Propylthio, 1-Methylethylthio, Butylthio, 1-Methylpropylthio, 2-Methylpropylthio oder 1,1-Dimethylethylthio;
- C₁-C₄-Alkylsulfinyl (C₁-C₄-Alkyl-S(=O)-): z.B. für Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₄-Alkylsulfonyl (C₁-C₄-Alkyl-S(=O)₂-): z. B. für Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl;
- Phenyl-C₁-C₄-alkyl: z. B. für Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmeth)-eth-1-yl, 1-(Phenylmethyl)-1-(methyl)-eth-1-yl oder -(Phenylmethyl)-1-(methyl)-prop-1-yl; vorzugsweise Benzyl;
- 3- bis 8-gliedriges Heterocyclyl: ein heterocyclischer Rest, der 3, 4, 5, 6, 7 oder 8 Ringglieder aufweist, wobei 1, 2 oder 3 der Ringglieder Heteroatome sind, die ausgewählt sind unter Sauerstoff, Schwefel, Stickstoff und einer Gruppe NR⁷ (worin R⁷ für Wasserstoff, C₁-C₆-Alkyl, C₃-C₆-Alkenyl oder C₃-C₆-Alkinyl steht). Außerdem kann der Heterocyclus gegebenenfalls ein oder zwei Carbonylgruppen oder Thiocarbonylgrupen als Ringglieder aufweisen. Der Heterocyclus kann aromatisch (Heteroaryl) oder teilweise oder vollständig gesättigt sein.

Beispiele für gesättigte Heterocyclen sind:
Oxiran-1-yl, Aziridin-1-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1, 3-Dioxepan-4-yl, 1,3-Dioxepan-5-yl, 1,3-Dioxepan-6-yl, 1,3-Dithiepan-2-yl, 1,3-Dithiepan-4-yl, 1,3-Dithiepan-5-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl;

Beispiele für ungesättigte Heterocyclen sind:
Dihydrofuran-2-yl, 1,2-Oxazolin-3-yl, 1,2-Oxazolin-5-yl, 1,3-Oxazolin-2-yl;

Beispiele für aromatisches Heterocyclyl sind die 5- und 6-gliedrigen aromatischen, heterocyclischen Reste, z.B. Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Furanyl und Thienyl.

In einer besonders bevorzugten Ausführungsform des erfindungsgemäßen Verfahrens weisen die Variablen R¹, R² und R³ die folgenden Bedeutungen, und zwar jeweils für sich allein oder in Kombination, auf:
- R¹: Wasserstoff, Amino oder C₁-C₄-Alkyl, insbesondere Wasserstoff, Amino, Methyl oder Ethyl;
- R²: Wasserstoff, C₁-C₄-Alkyl oder C₁-C₄-Halogenalkyl, insbesondere Wasserstoff, Methyl, Difluormethyl, Difluorchlormethyl oder Trifluormethyl;
- R³: Wasserstoff;

In einer weiteren besonders bevorzugten Ausführungsform des erfindungsgemäßen Verfahrens stehen X¹, X² und X³ jeweils für Sauerstoff.

Die Gruppe Ar steht bevorzugt für eine Gruppe der Formel Ar-1 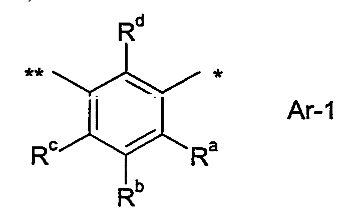 worin
- R^{a}, R^{b}, R^{c} und R^{d}: unabhängig voneinander für Wasserstoff, Halogen, C₁-C₄-Alkyl, C₁-C₄-Halogenalkyl oder Cyano stehen;
- *: die Verknüpfung von Ar mit der C(X³)-Gruppe kennzeichnet; und
- **: die Verknüpfung von Ar mit dem Stickstoffatom der (Thio)uracil-, Dithiouracilrestes beziehungsweise der Iso(thio)cyanato-Gruppe kennzeichnet.

In einer besonders bevorzugten erfindungsgemäßen Ausführungsform weisen die Variablen R^{a}, R^{b}, R^{c} und R^{d} jeweils für sich allein oder in Kombination die folgenden Bedeutungen auf:
- R^{a}: Halogen, Cyano oder C₁-C₄-Halogenalkyl,
insbesondere Fluor, Chlor, Cyano oder Trifluormethyl;
- R^{b}, R^{d}: jeweils Wasserstoff;
- R^{c}: Wasserstoff oder Halogen,
insbesondere Fluor, Chlor oder Wasserstoff.

Der von einem primären oder sekundären Amin abgeleitete Rest A steht in der Regel für eine Gruppe der Formel -NR⁵R⁶,
worin die Variablen R⁵ und R⁶ unabhängig voneinander die folgenden Bedeutungen aufweisen:
- R⁵, R⁶: Wasserstoff, C₁-C₁₀-Alkyl, C₂-C₁₀-Alkenyl oder C₂-C₁₀-Alkinyl, die unsubstituiert oder durch einen der folgenden Reste substituiert sein können: C₁-C₄-Alkoxy, C₁-C₄-Alkylthio, CN, NO₂, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, 3- bis 8-gliedriges Heterocyclyl mit ein bis drei Heteroatomen ausgewählt unter O, S, N und einer Gruppe NR⁷,
worin R⁷ für Wasserstoff, C₁-C₆-Alkyl, C₃-C₆-Alkenyl oder C₃-C₆-Alkinyl steht,
Phenyl, das seinerseits 1, 2, 3 oder 4 Substituenten ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, C₁-C₃-Alkylamino, C₁-C₃-Dialkylamino, Formyl, Nitro oder Cyano aufweisen kann;
C₁-C₁₀-Halogenalkyl, C₂-C₁₀-Halogenalkenyl, C₂-C₁₀-Halogenalkinyl, C₃-C₈-Cycloalkyl, C₃-C₁₀-Cycloalkenyl, 3- bis 8-gliedriges Heterocyclyl mit ein bis drei Heteroatomen ausgewählt unter O, S, N und einer Gruppe NR⁷,
worin R⁷ für Wasserstoff, C₁-C₆-Alkyl, C₃-C₆-Alkenyl oder C₃-C₆-Alkinyl steht,
Phenyl oder Naphthyl,
wobei C₃-C₈-Cycloalkyl, C₃-C₁₀-Cycloalkenyl, 3- bis 8-gliedriges Heterocyclyl, Phenyl oder Naphthyl ihrerseits 1, 2, 3 oder 4 Substituenten ausgewählt unter
Halogen, C₁-C₄-Alkyl, C₁-C₄-Alkoxy, C₁-C₄-Fluoralkyl, C₁-C₄-Alkyloxycarbonyl, Trifluormethylsulfonyl, Formyl, C₁-C₃-Alkylamino, C₁-C₃-Dialkylamino, Phenoxy, Nitro oder Cyano aufweisen können; oder
- R⁵ und R⁶: bilden gemeinsam einen gesättigten oder teilweise ungesättigten 5- bis 8-gliedrigen Stickstoffheterocyclus, der ein oder zwei Carbonylgruppen, Thiocarbonylgruppen und/oder ein oder zwei weitere Heteroatome ausgewählt unter O, S, N und einer Gruppe NR⁷,
worin R⁷ für Wasserstoff, C₁-C₆-Alkyl, C₃-C₆-Alkenyl oder C₃-C₆-Alkinyl steht,
als Ringglieder aufweisen kann; und der durch C₁-C₄-Alkyl, C₁-C₄-Alkoxy und/oder C₁-C₄-Halogenalkyl substituiert sein kann.

Bevorzugte Substituenten R⁵ und R⁶ sind unabhängig voneinander Wasserstoff, C₁-C₆-Alkyl,
das gegebenenfalls durch einen Substituenten ausgewählt unter Halogen, Cyano, C₁-C₄-Alkoxy, C₁-C₄-Alkoxycarbonyl, C₁-C₄-Alkylthio, C₃-C₈-Cycloalkyl, Phenyl,
das seinerseits gegebenenfalls durch Halogen oder C₁-C₄-Alkoxy substituiert ist,
Furyl, Thienyl oder 1,3-Dioxolanyl
substituiert ist.

Bevorzugte Substituenten R⁵ und R⁶ sind weiterhin C₂-C₆-Alkenyl, C₂-C₆-Alkinyl, C₃-C₈-Cycloalkyl oder Phenyl,
das gegebenenfalls durch 1 oder 2 Substituenten, ausgewählt unter Halogen, C₁-C₄-Alkyl, C₁-C₄-Fluoralkyl, C₁-C₄-Alkoxy, C₁-C₄-Alkoxycarbonyl, Nitro oder C₁-C₃-Dialkylamino substituiert ist,
Naphthtyl oder Pyridyl.

In einer weiteren bevorzugten Ausführungsform bilden R⁵ und R⁶ zusammen einen fünf-, sechs- oder siebengliedrigen gesättigten oder ungesättigten Stickstoffheterocyclus, der ein weiteres Heteroatom ausgewählt unter N, O, und einer Gruppe NR⁷, worin R⁷ für Wasserstoff, C₁-C₆-Alkyl, C₃-C₆-Alkenyl oder C₃-C₆-Alkinyl steht
als Ringglied enthalten kann, und/oder
durch ein, zwei oder drei Substituenten ausgewählt unter C₁-C₄-Alkyl und C₁-C₄-Halogenalkyl substituiert sein kann.

In einer besonders bevorzugten Ausführungsform des erfindungsgemäßen Verfahrens steht einer der Reste R⁵ oder R⁶ für Wasserstoff, C₁-C₆-Alkyl , C₂-C₆-Alkenyl oder C₂-C₆-Alkinyl und der andere Rest R⁵ oder R⁶ für C₁-C₆-Alkyl, C₃-C₈-Cycloalkyl oder Phenyl.

Demnach betrifft die vorliegende Erfindung insbesondere ein Verfahren zur Herstellung der 3-Phenyl(thio)uracile oder 3-Phenyldithiouracile der Formel I, worin Ar für Ar-1 steht. Diese Verbindungen werden im Folgenden als IA bezeichnet:

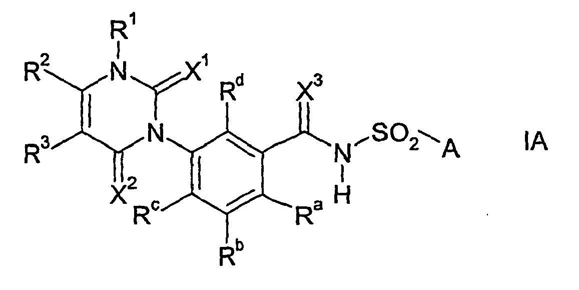

Die Variablen R¹, R², R³, R^{a}, R^{b}, R^{c}, R^{d}, X¹, X², X³ und A weisen die zuvor genannten Bedeutungen auf.

Das Verfahren umfasst bevorzugt die Umsetzung eines Phenyliso(thio)cyanats der Formel IIA 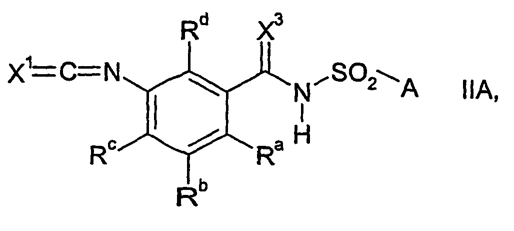 worin
- X¹, X³: jeweils unabhängig voneinander Sauerstoff oder Schwefel;
- R^{a}, R^{b}, R^{c} und R^{d}: jeweils unabhängig voneinander
Wasserstoff, Halogen, Cyano, C₁-C₄-Alkyl oder C₁-C₄-Halogenalkyl bedeuten; und
- A: die zuvor genannte Bedeutung aufweist;
insbesondere für eine Gruppe NR⁵R⁶ steht,
worin R⁵ und R⁶ die zuvor genannten Bedeutungen,
insbesondere die als bevorzugt angegebenen
beziehungsweise die als besonders bevorzugt angegebenen Bedeutungen aufweist.

Insbesondere betrifft die vorliegende Erfindung ein Verfahren zur Herstellung der 3-Phenyl(thio)uracile oder 3-Phenyldithiouracile IA,
worin A für NR⁵R⁶ steht; und
die Variablen R¹, R², R³, R^{a}, R^{b}, R^{c}, R^{d}, X¹, X² und X³ unabhängig voneinander, vorzugsweise in Kombination miteinander, die folgenden Bedeutungen aufweisen:
- R¹: Wasserstoff, Amino oder C₁-C₄-Alkyl,
insbesondere Wasserstoff, Amino, Methyl oder Ethyl;
- R²: Wasserstoff, C₁-C₄-Alkyl oder C₁-C₄-Halogenalkyl,
insbesondere Wasserstoff, Methyl, Difluormethyl, Difluorchlormethyl oder Trifluormethyl;
- R³: Wasserstoff;
- R^{a}: Halogen, Cyano oder C₁-C₄-Halogenalkyl,
insbesondere Fluor, Chlor, Cyano oder Trifluormethyl;
- R^{b}, R^{d}: Wasserstoff;
- R^{c}: Wasserstoff oder Halogen,
insbesondere Fluor, Chlor oder Wasserstoff;
- X¹, X² und X³: jeweils Sauerstoff.

Das erfindungsgemäße Verfahren umfasst die Umsetzung eines Phenyliso(thio)-cyanats der Formel II mit einem Enamin der Formel III zu 3-Phenyl(thio)uracilen oder 3-Phenyldithiouraclilen der Formel I mit R¹=R^{1a}; und
gegebenenfalls in einem weiteren Schritt die Umsetzung des erhaltenen 3-Phenyl-(thio)uracils oder 3-Phenyldithiouracils der Formel I mit R¹-R^{1a}, wenn R¹ für Wasserstoff steht, mit einem Aminierungsmittel der Formel IV zu 3-Phenyl(thio)uracilen oder 3-Phenyldithiouracilen der Formel I mit R¹=Amino:

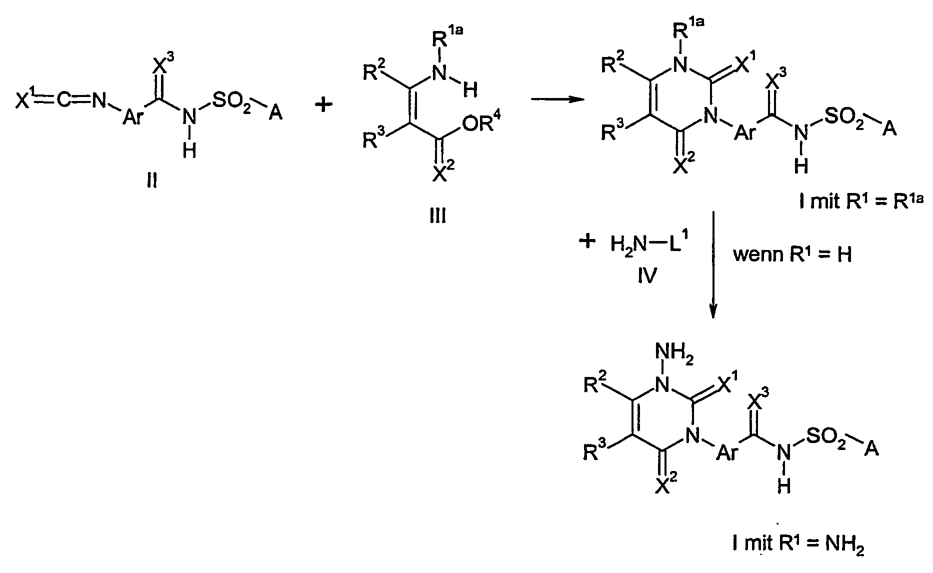

In der Regel erfolgt die Umsetzung des Enamins III mit dem Phenyliso(thio)cyanat II in Gegenwart einer Base.

Als Base kommen alle üblichen organischen und anorganischen Basen in Betracht.

Geeignete anorganische Basen umfassen beispielsweise Alkali- und Erdalkalimetallcarbonate wie Lithiumcarbonat, Natriumcarbonat, Kaliumcarbonat, Cäsiumcarbonat, Calciumcarbonat, Alkali- oder Erdalkalimetallhydride wie Lithiumhydrid, Natriumhydrid, Kaliumhydrid oder Calciumhydrid.

Geeignete organische Basen umfassen Alkali- und Erdalkalimetallalkoholate wie Lithiummethylat, Natriummethylat, Kaliummethylat, Calciummethylat, Lithiumethylat, Natriumethylat, Kaliumethylat, Calciumethylat, Natrium-n-propylat oder -isopropylat, Kalium-n-propylat oder -isopropylat, Natrium-n-butylat , -iso-butylat, -sec-butylat oder - tert-butylat, Kalium-n-butylat, -iso-butylat, -sec-butylat oder -tert-butylat, Natrium-n-pentylat, -isopentylat, -sec-pentylat oder -tert-pentylat, Kalium-n-pentylat, -isopentylat, -sec-pentylat- oder -tert-pentylat (=-tert.-amylat), tertiäre Amine wie Tributylamin, Pyridin, 1,8-Diazabicyclo[5.4.0]undec-7-en (DBU), 1,5-Diazabicyclo[4.3.0]-non-5-en (DBN) oder 1,4-Diazabicyclo[2.2.2]-octan (DABCO).

Geeignete Basen sind weiterhin lithiumorganische Verbindungen wie n-Butyllithium, sec-Buthyllithium, Phenyllithium sowie Alkalimetallamide wie Lithiumdiisopropylamid und Natrium(bis(trimethylsilyl))amid, Geeignet sind außerdem Cäsiumfluorid sowie Alkali- und Erdalkalimetallhydroxide wie Natriumhydroxid, Kaliumhydroxid, Lithiumhydroxid, Calciumhydroxid. Bevorzugte Basen sind Alkalimetallalkoholate, insbesondere Lithium, Natrium- und Kaliumalkoholate der oben genannten C₁-C₅-Alkanole, die vorgenannten Alkalimetallhydride, Alkalimetallcarbonate und Amidinbasen.

In einer besonders bevorzugten Ausführungsform des erfindungsgemäßen Verfahrens setzt man Natriumhydrid oder Kaliummethylat als Base ein.

In der Regel setzt man 1,8 bis 2,6 Basenäq uivalente pro Mol des Phenyliso(thio)cyanats der Formel II ein.

Das Enamin der Formel III kann substöchiometrisch, äquimolar oder überstöchiometrisch bezogen auf das Phenyliso(thio)cyanat der Formel II eingesetzt werden. In der Regel setzt man pro Mol des Phenyliso(thio)cyanats der Formel II 0,9 bis 1,3 Mol, vorzugsweise 0,95 bis 1,15 Mol, Enamin der Formel III ein.

Die Umsetzung eines Phenyliso(thio)cyanats der Formel II mit dem Enamin der Formel III erfolgt üblicherweise in einem Lösungsmittel oder Verdünnungsmittel. Hierfür kommen alle inerten, organischen Lösungsmittel oder Lösungsmittelgemische in Betracht. Für diese Umsetzungen verwendet man als Lösungsmittel - je nach Temperaturbereich - aliphatische, cycloaliphatische oder aromatische Kohlenwasserstoffe wie Pentan, Hexan, Cyclopentan, Cyclohexan, Toluol, Xylol, chlorierte Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Ether wie Tetrahydrofuran, 1,4-Dioxan, Anisol, Glykolether wie Dimethylglykolether, Diethylglykolether, Diethylenglykoldimethylether, Ester wie Methylacetat, Ethylacetat, Propylacetat, n-Butylacetat, Methylisobutyrat, Isobutylacetat, Carbonate wie Dimethylcarbonat, Diethylcarbonat und Ethylencarbonat, Carbonsäureamide wie N,N-Dimethylformamid, N,N-Dimethylacetamid, N-Methylpyrrolidon, Nitrokohlenwasserstoffe wie Nitrobenzol, Tetraalkylhamstoffe wie Tetraethylharnstoff, Tetrabutylharnstoff, Dimethylethylenharnstoff, Dimethylpropylenharnstoff, Nitrile wie Acetonitril, Propionitril, Butyronitril oder Isobutyronitril, Sulfoxide wie Dimethylsulfoxid oder auch Gemische der genannten Lösungsmittel.
Sofern die Base Lösungsmitteleigenschaften hat, wie im Falle des Pyridins und des Tributylamins, kann auch die Base, bzw. eine Mischung der Base mit einem der vorgenannten Lösungsmittel, als Lösungs- bzw. Verdünnungsmittel für die Umsetzung von II mit III eingesetzt werden.

Besonders bevorzugt ist ein aprotisches, polares Lösungsmittelsystem, das auch Gemische verschiedener aprotischer, polarer Lösungsmittel und Gemische verschiedener aprotischer polarer Lösungsmittel mit aprotischen unpolaren Lösungsmitteln umfasst. Der Anteil an polarem, aprotischen Lösungsmittel beträgt in derartigen Lösungsmittelsystemen wenigstens 50 Vol.-%, bevorzugt wenigstens 75 Vol-%, insbesondere wenigstens 85 Vol.-%. Bevorzugte aprotische, polare Lösungsmittel sind die genannten N,N-Dimethylamide aliphatischer C₁-C₄-Carbonsäuren wie N,N-Dimethylformamid und N,N-Dimethylacetamid, N-Methyllactame wie N-Methylpyrrolidon, Carbonate wie Dimethylcarbonat, Diethylcarbonat und Ethylencarbonat, Nitrile wie Acetonitril, Propionitril, Butyronitril und Isobutyronitril, Sulfoxide wie Dimethylsulfoxid, Cyclische Ether wie Tetrahydrofuran und Dioxan, Ester wie Ethylacetat, n-Butylacetat oder Gemische davon und hiervon bevorzugt die Dimethylcarbonsäureamide.

In einer bevorzugten Ausführungsform verwendet man wenigstens ein aprotisches, polares Lösungsmittel als alleiniges Lösungsmittelsystem (mehr als 99 Vol.-%, bezogen auf die Gesamtvolumenmenge), beispielsweise ein Gemisch aus N,N-Dimethylformamid und Tetrahydrofuran. In einer weiteren bevorzugten Ausführungsform der Erfindung setzt man ein Lösungsmittelsystem ein, das neben dem aprotischen, polaren Lösungsmittel, insbesondere neben dem besonders bevorzugten aprotischen, polaren Lösungsmittel, 0,5 bis 25 Vol.-% wenigstens eines aprotischen, unpolaren Lösungsmittels, insbesondere wenigstens eines aromatischen oder aliphatischen Kohlenwasserstoffes, speziell Toluol oder Hexan umfasst. Dementsprechend ist der Anteil an aprotischem, polaren Lösungsmittel in dieser Mischung 75,0 bis 99,5 Vol.-%. Bevorzugte aprotische unpolare Lösungsmittel sind aliphatische Kohlenwasserstoffe wie n-Hexan, Isohexan (kommerzielles Hexan-Gemisch), n-Heptan, Dekan, Petrolether, cycloaliphatische Kohlenwasserstoffe wie Cyclohexan und aromatische Kohlenwasserstoffe wie Toluol, Benzol oder Xylol.

Untersuchungen haben gezeigt, dass die Ausbeute an Wertprodukt I durch die Anwesenheit von Wasserspuren in dem Reaktionsgemisch beeinträchtigt wird. In einer bevorzugten Ausführungsform des erfindungsgemäßen Verfahrens trocknet man die eingesetzten Materialien so weit, dass der Gehalt an Wasser im aprotischen, polaren Lösungsmittel nicht mehr als 0,5 Gew.-%, häufig nicht mehr als 0,2 Gew.-%, vorzugsweise nicht mehr als 0,05 und insbesondere nicht mehr als 0,02 Gew.-% Wasser, bezogen auf die Gesamtmenge an Edukt II, Edukt III und Lösungsmittel beträgt. Die quantitative Bestimmung von Wasser kann chemisch, beispielsweise durch Karl-Fischer-Titration oder physikalisch, beispielsweise durch Bestimmung der Dielektrizitätskonstanten oder quantitative HPLC erfolgen.

In einer bevorzugten Ausführungsform umfasst das erfindungsgemäße Verfahren daher auch eine Vorbehandlung des Lösungs- oder Verdünnungsmittels und/oder der Edukte zur Trocknung der eingesetzten Chemikalien. Verfahren zum Trocknen von Lösungsmitteln sind dem Fachmann auf dem Gebiet der organischen Synthese bekannt, beispielsweise durch Verwendung von Trockenmitteln.

Ein bevorzugtes Verfahren umfasst das Trocknen durch azeotrope Trocknung. Bei der azeotropen Trocknung versetzt man den zu trocknenden Stoff mit einer Chemikalie, die mit Wasser ein Azeotrop bildet (Schleppmittel) und entfernt anschließend das wasserhaltige Azeotrop auf destillativem Weg. Üblicherweise handelt es sich bei dem Schleppmittel um ein organisches Lösungsmittel. Beispiele hierfür sind Kohlenwasserstoffe wie Benzol, Toluol, Xylol, Pentan oder Hexan, Chloraromaten wie Chlorbenzol und Alkylester aliphatischer Carbonsäuren wie Ethylacetat und n-Butylacetat.

In einer bevorzugten Ausführungsform legt man das Enamin III in dem aprotischen, polaren Lösungs- oder Verdünnungsmittel, beispielsweise N,N-Dimethylformamid, vor. Anschließend gibt man 20 bis 200 Vol.-%, vorzugsweise 50 bis 150 Vol.-% und insbesondere 80 bis 130 Vol.-%, bezogen auf das aprotisch polare Lösungs- bzw. Verdünnungsmittel, eines hierfür.geeigneten Schleppmittels zu und trocknet azeotrop. Die erforderliche Trocknungsdauer hängt naturgemäß vom Wassergehalt der eingesetzten Stoffe, von der Ansatzgröße und von den verwendeten Apparaturen ab und kann vom Fachmann durch Routinemethoden ermittelt werden. Anschließend setzt man das Enamin III mit dem Phenyliso(thio)cyanat II auf die nachfolgend beschriebene Weise um.

Bei dem erfindungsgemäßen Verfahren können die Edukte und Reagenzien grundsätzlich in beliebiger Reihenfolge zusammengegeben werden, d. h. die Reaktanden und die Base können getrennt, gleichzeitig oder nacheinander in das Reaktionsgefäß eingebracht und zur Reaktion geführt zu werden. Bei allen Verfahrensvarianten setzt man vorzugsweise das Enamin III, die Base und/oder das Phenyliso(thio)cyanat II verdünnt in einem der vorgenannten Lösungsmittel oder Lösungsmittelgemische ein.

Vorteilhaft legt man das Enamin III vor und gibt unter Durchmischen, z. B. Rühren, die Base in den Reaktionsansatz. Die Reaktionstemperatur bei der Basenzugabe richtet sich nach der Reaktivität der eingesetzten Base. In der Regel liegen sie in einem Bereich von -20 °C bis 80 °C. Vorteilhaft rührt man zur Vervollständigung der Reaktion noch bei der gleichen Temperatur oder höherer Temperatur nach. Die erforderlichen Reaktionszeiten kann der Fachmann anhand von Routinemethoden ermitteln.

Vorteilhaft gibt man die Base zu dem Enamin III. In der Regel erfolgt die Zugabe der Base unter Temperaturkontrolle. Beispielsweise gibt man die Alkali- oder Erdalkalimetallhydride zu dem Enamin III bevorzugt in einem Temperaturbereich von -20 °C bis 20 °C und rührt in diesem Temperaturbereich zur Vervollständigung der Deprotonierung des Enamins nach. Bei Verwendung von Alkali- oder Erdalkalimetallcarbonaten erfolgt die Basenzugabe zu dem Enamin III in der Regel bei Temperaturen von nicht mehr als 50°C, insbesondere nicht mehr als 45°C, z.B. im bereich von 20°C bis 50 °C und man rührt bei Temperaturen bis 80 °C, z. B. 35 bis 80°C nach. Bei Verwendung von Alkali- und Erdalkalimetallalkoholaten erfolgt die Basenzugabe insbesondere bei Temperaturen von -20 °C bis 50 °C, vorteilhaft -15 ° C bis 20 °C und man rührt bei Temperaturen von -10 °C bis 80 °C nach. Danach gibt man das Phenyliso(thio)cyanat II zu und führt die Reaktion zu Ende.

Selbstverständlich kann man auch die Base im Falle der Verwendung von Alkali- oder Erdalkalimetallalkoholaten oder -carbonaten in einem getrockneten, polaren Lösungsmittel vorlegen, danach das Enamin III in einem der zuvor genannten, getrockneten polaren Lösungsmittel oder Lösungsmittelgemische zugeben oder wie zuvor beschrieben azeotrop trocknen und dann das Phenyliso(thio)cyanat II zugegeben. Alternativ kann man auch die Verbindungen II und III als Gemisch in einem der zuvor genannten, getrockneten polaren Lösungsmittel oder Lösungsmittelgemische vorlegen und anschließend die Base in einem der zuvor genannten, getrockneten Lösungsmittel oder. Lösungsmittelgemische zugeben. In einer weiteren Variante des erfindungsgemäßen Verfahrens legt man die Base in dem zuvor genannten, getrockneten Lösungsmittel oder Lösungsmittelgemische vor und gibt danach ein Gemisch aus Verbindung II und III in einem der zuvor genannten, getrockneten Lösungsmittel oder Lösungsmittelgemische zu.

Die Reaktion wird vorzugsweise so durchgeführt, dass man die Base zu dem Enamin der Formel III in einem der vorgenannten getrockneten Lösungsmittel oder Lösungsmittelgemische gibt. Nach dem Nachrühren fügt man das Phenyliso(thio)cyanat II in einem der vorgenannten Lösungsmittel oder Lösungsmittelgemische zu und lässt noch nachreagieren.

Die Reaktionstemperatur für die Umsetzung des Phenyliso(thio)cyanats II mit dem Enamin III in Gegenwart einer Base liegt in der Regel in einem Bereich von -20 bis 80 °C.

Bei Verwendung von Alkali- oder Erdalkalimetallhydriden als Base gibt man in der Regel das Phenyliso(thio)cyanat II bei einer Temperatur von -20 °C bis 20 °C, vorzugsweise -5 bis 10 °C zu dem Gemisch aus Base und Enamin III und rührt danach bei Temperaturen bis 50°C, z.B. 20 bis 50 °C nach.

Bei Verwendung von Alkali- und Erdalkalimetallalkoholaten gibt man üblicherweise das Phenyliso(thio)cyanat II bei einer Temperatur von -20 °C bis 20 °C, vorzugsweise -15 °C bis 10 °C zu dem Gemisch aus Base und Enamin III und rührt bei Temperaturen bis 80°C, z.B. 0 bis 80°C nach.

Bei Verwendung von Alkali- und Erdalkalimetallcarbonaten erfolgt üblicherweise die Zugabe des Phenyliso(thio)cyanates II zu dem Gemisch aus Base und Enamin III bei Temperaturen bis 50°C, z.B. 20 bis 50°C und zur Vervollständigung der Umsetzung rührt man anschließend bei Temperaturen bis 80 °C, z.B. 20 bis 80°C, vorzugsweise 40 bis 80°C nach. Die zur Erreichung des gewünschten Umsatzes erforderliche Reaktionszeit kann der Fachmann durch Routinemethoden ermitteln.

Die Reaktion kann bei Normaldruck, sowie bei Unterdruck oder unter erhöhtem Druck, kontinuierlich oder diskontinuierlich durchgeführt werden. In der Regel ist es von Vorteil, die Umsetzung unter einer Schutzgasatmosphäre wie Stickstoff durchzuführen.

Die Aufarbeitung zur Gewinnung des Zielproduktes kann nach den hierfür üblichen Verfahren erfolgen. In der Regel wird man hierzu die basische Reaktionsmischung durch Zugabe von Säure auf einen pH-Wert ≤4, insbesondere ≤2 einstellen und anschließend durch Zugabe von Wasser eine Kristallisation oder Fällung der Verbindung I bewirken. Zugabe von Säure und Wasser können auch gleichzeitig erfolgen, beispielsweise durch Zugabe einer verdünnten wässrigen Säuren. Grundsätzllich kann man das Reaktionsgemisch auch wässrig-extraktiv aufarbeiten, z.B. indem man nach Neutralisation der alkalischen Reaktionsmischung diese, gegebenenfalls nach Entfernen der Hauptmenge des Lösungsmittels, zwischen Wasser und einem mit Wasser nicht mischbaren organischen Lösungmittel verteilt und anschließend die Verbindung I aus der organischen Phase isoliert. Diesen Methoden können sich weitere Schritte zur Reinigung, z.B. Fällung, Kristallisation und/oder extraktive Schritte, anschließen.

Die zur Durchführung des erfindungsgemäßen Verfahrens als Edukte benötigten Enamine der Formel III sind bekannte Verbindungen und/oder lassen sich in Analogie zu bekannten Verfahren herstellen (z.B. A. Lutz, A. und S. Troto, J. of Heterocyclic Chem. 1972, 9, 3, 513-522).

Die Phenyliso(thio)cyanate der Formel II sowie Verfahren zu deren Herstellung sind Gegenstand der älteren deutschen Patentanmeldung 102 50 614.0, auf deren Offenbarung hiermit Bezug genommen wird. Dieses Verfahren umfasst die Umsetzung einer Verbindung VI, 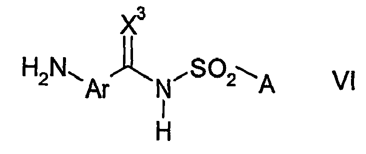 worin X³, Ar und A die zuvor genannten Bedeutungen aufweisen, mit einem Phosgenierungsmittel wie Phosgen, Thiophosgen oder Diphosgen, in hoher Ausbeute und Reinheit.

Die Umsetzung von Verbindung VI mit dem Phosgenierungsmittel erfolgt üblicherweise in einem inerten organischen Lösungsmittel. Als Lösungsmittel verwendet man für diese Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, n-Butylacetat, Methylisobutyrat, Isobutylacetat, Carbonsäureamide wie N,N-Dimethylformamid, N-Methylpyrrolidon, Carbonate wie Dimethylcarbonat, Diethylcarbonat, Ethylencarbonat, Nitrokohlenwasserstoffe wie Nitrobenzol, Tetraalkylhamstoffe wie Tetraethylharnstoff, Tetrabutylharnstoff, Dimethylethylenharnstoff, Dimethylpropylenharnstoff, Nitrile wie Acetonitril, Propionitril, Butyronitril oder Isobutyronitril oder auch Gemische einzelner Lösungsmittel.

Bei Verwendung von Phosgen wird man vorzugsweise ein Lösungsmittel einsetzen, das weitgehend von protischen Verunreinigungen wie Wasser und Alkoholen befreit ist. Bei der Herstellung der Isothiocyanate kann man jedoch auch in Anlehnung an Houben-Weyl, Methoden der organischen Chemie, 4. Auflage, Bd. IX, S. 875, die Umsetzung von II mit Thiophosgen in einem Zweiphasensystem aus Wasser und einem damit nicht mischbaren organischen Lösungsmittel oder auch in Wasser durchführen.

Die Reaktionstemperatur wird in der Regel 180 °C, vorzugsweise 120 °C und insbesondere 100 °C nicht überschreiten und wird in der Regel wenigstens 40 °C und vorzugsweise wenigstens 50 °C betragen. Häufig wird man so vorgehen, dass man zumindest die Hauptmenge des Phosgenierungsmittels bei einer niedrigen Temperatur, z. B. im Bereich von 0 bis 40 °C, insbesondere 10 bis 40 °C und speziell 20 bis 30 °C zugibt und während oder nach beendeter Zugabe auf eine Temperatur im Bereich von 40 bis 180 °C, insbesondere 50 bis 120 °C und speziell 70 bis 100 °C erwärmt bis der Umsatz vollständig ist.

In der Regel setzt man 0,9 bis 2, vorzugsweise 0,95 bis 1,5, besonders bevorzugt 0,98 bis 1,09 Moläquivalente Phosgenierungsmittel pro Mol der Verbindung VI ein.

Gegebenenfalls führt man die Umsetzung von VI in Gegenwart einer Base durch. Als Basen kommen beispielsweise basische anorganische Verbindungen in Betracht, z. B. Alkali- oder Erdalkalihydroxide, -hydrogencarbonate oder -carbonate. Man kann die Reaktion jedoch auch in Gegenwart einer organischen Base, beispielsweise eines tiertiären Amins wie Triethylamin, Tri-n-propylamin, N-Ethyldiisopropylamin, Tri-n-butylamin, Pyridin, α-, β-, γ-Picolin, 2,4-, 2,6-Lutidin, N-Methylpyrrolidin, Dimethylanilin, N,N-Dimethylcyclohexylamin, Chinolin oder Acridin durchführen. Die Base (berechnet als Basenäquivalent) kann substöchiometrisch, überstöchiometrisch oder äquimolar, bezogen auf die Verbindung VI, eingesetzt werden. Pro Mol der Verbindung VI setzt man im allgemeinen 0,01 bis 6 Mol, vorzugsweise 0,1 bis 3 Mol Base ein.

In einer anderen Ausführungsform des Verfahrens führt man die Umsetzung in Gegenwart von Chlorwasserstoff durch. Die Menge an Chlorwasserstoff beträgt dann üblicherweise 0,9 bis 5,0 mol, vorzugsweise 1,0 bis 2,5 mol und insbesondere 1,0 bis 1,2 mol Chlorwasserstoff pro Mol der Verbindung VI. Hierbei wird man in der Regel so vorgehen, dass man zunächst in eine Lösung oder Suspension die Verbindung VI in einem der vorgenannten Lösungsmittel die vorgenannte Menge an gasförmigen Chlorwasserstoff einleitet oder eine Lösung von Chlorwasserstoff in einem Lösungsmittel zugibt, dann das Phosgenierungsmittel in der zuvor beschriebenen Weise zugibt und die Reaktion dann in der oben beschriebenen Weise fortführt. Das Einleiten von Chlorwasserstoff erfolgt üblicherweise bei Temperaturen zwischen 10 °C und 60 °C, vorzugsweise bei 20 bis 30 °C.

Sofern man das Verfahren in Gegenwart von Chlorwasserstoff durchführt, kann Aktivkohle als Katalysator verwendet werden. Zweckmäßigerweise beträgt die Menge an Aktivkohle 1 bis 10 Gew.-%, vorzugsweise 1 bis 3 Gew.-%, bezogen auf das Gewicht der Verbindung VI.

Die Verbindungen der allgemeinen Formel VI sind ebenfalls aus der älteren deutschen Patentanmeldung DE 102 50 614.0 bekannt. Die Verbindungen der Formel VI können in Analogie zu bekannten Verfahren zur Herstellung von Anilinen gewonnen werden.

Die Anilinverbindungen der Formel VI kann man beispielsweise gemäß Schema 1 herstellen, indem man zunächst eine Aroylverbindung der Formel VII mit einem Sulfamidsäureamid VIII im Sinne einer Kondensationsreaktion zu einem N-Aroylsulfamidsäureamid der allgemeinen Formel IX umsetzt und anschließend das erhaltene N-Aroylsulfamidsäureamid IX zur Verbindung VI reduziert.

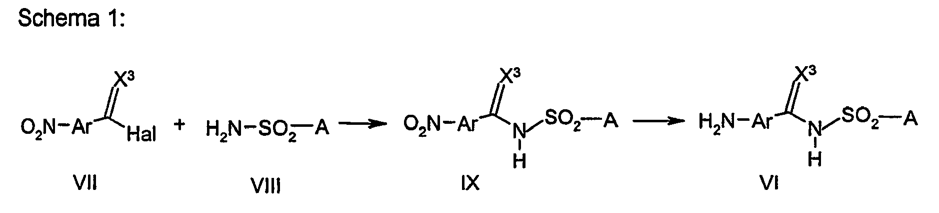

In Schema 1 haben die Variablen A, Ar und X³ die vorgenannten Bedeutungen. Die Kondensation von Aroylverbindungen der allgemeinen Formel VII mit Sulfamidsäureamiden der allgemeinen Formel VIII zu den entsprechenden Benzoylsulfamiden der allgemeinen Formel IX erfolgt in Anlehnung an bekannte Verfahren, beispielsweise wie in der WO 01/83459, S. 31-35, in der PCT/EP 03/05126 beschrieben, auf deren Offenbarung hiermit Bezug genommen wird.

Die Reduktion der Nitroverbindung IX zum Anilin VI gelingt beispielsweise mit nascierendem Wasserstoff. Hierzu setzt man die Nitroverbindung IX mit einer Säure in Gegenwart eines unedlen Metalls um. Unedle Metalle sind naturgemäß solche, die von einer Brönstedsäure unter Wasserstoffentwicklung gelöst werden. Derartige Metalle weisen in der Regel ein Normalpotential < 0 V und insbesondere kleiner gleich -0,1 V, z. B. im Bereich von -0,1 bis -1,0 V (in saurer wässriger Lösung bei 15 °C und 1 bar) auf. Beispiele für geeignete Metalle sind Zn, Fe und Sn, insbesondere Fe. Als Säuren kommen für diesen Zweck sowohl anorganische Mineralsäuren, beispielsweise Salzsäure oder verdünnte Schwefelsäure, oder Mischungen aus anorganischer Säure und einem inerten Lösungsmittel, beispielsweise gasförmige HCl in einem Ether oder einem Alkohol oder in einer Mischung davon, oder organische Carbonsäuren, zweckmäßig Essigsäure, Propionsäure oder Buttersäure, in Betracht.

Als Reduktionsmittel kommen weiterhin auch Metallhydride und Halbmetallhydride wie Aluminiumhydrid und davon abgeleitete Hydride wie Lithiumaluminiumhydrid, Diisobutylaluminiumhydrid, Borhydride wie Diboran und davon abgeleitete Boranate wie Natriumborhydrid oder Lithiumboranat in Betracht. Hierzu bringt man die Nitroverbindung IX in einem inerten Lösungsmittel mit dem komplexen Metallhydrid bei 10 bis 65 °C, vorteilhaft 20 bis 50 °C in Kontakt.

Ein weiteres geeignetes Reduktionsmittel für die Umwandlung der Verbindung IX in die Verbindung VI ist Wasserstoff in Gegenwart katalytischer Mengen an Übergangsmetallen oder Übergangsmetallverbindungen, insbesondere der 8. Nebengruppe.

Die Reduktion der Verbindung IX zur Verbindung VI kann auch mit Natriumsulfid, vorteilhaft in wässrig ammoniakalischer Lösung, in Gegenwart von Ammoniumchlorid gemäß dem in Org. Syn., Coll. Vol., 3, 82 (1955) beschriebenen Verfahren erfolgen.

Die in Schema 1 eingesetzten Aroylverbindungen VII sind nach an sich im Stand der Technik bekannten Verfahren erhältlich oder lassen sich in Anlehnung an bekannte Verfahren herstellen, beispielsweise gemäß US 6,251,829, EP 415 641, EP 908 457, EP 1176133 und WO 01/087872.

Das erfindungsgemäße Verfahren ermöglicht die Herstellung des Wertproduktes I in hoher Ausbeute und vorzüglicher Reinheit, so dass aufwändige Reinigungsverfahren nicht erforderlich sind. Das erfindungsgemäße Verfahren ist somit einfacher und wirtschaftlicher durchzuführen als die aus dem Stand der Technik bekannten Verfahren. Führt man die Umsetzung mit einem Enamin III durch, worin R^{1a} beispielsweise für C₁-C₆-Alkyl, insbesondere für Methyl steht, so erhält man direkt in hoher Ausbeute und Reinheit die in der WO 01/83459 beschriebenen Verbindungen.

Ein weiterer Gegenstand der Erfindung ist die Umsetzung der nach dem erfindungsgemäßen Verfahren erhaltenen Verbindungen I mit R¹ = Wasserstoff mit
(A) einem Aminierungsmittel der Formel IV

   H₂N-L¹ IV,

   worin
   - L¹: für eine nucleophil verdrängbare Abgangsgruppe steht,
   bevorzugt für Halogen, Hydrogensulfat, C₁-C₆-Alkylsulfonyloxy, C₁-C₆-Halogenalkylsulfonyloxy, Phenylsulfonyloxy oder Phenyloxy,
   wobei der Phenylring gegebenenfalls mit Halogen, Nitro, C₁-C₆-Alkyl oder C₁-C₆-Halogenalkyl ein- oder mehrfach substituiert ist,
   besonders bevorzugt für Halogen, Hydrogensulfat, C₁-C₆-Alkylsulfonyloxy, C₁-C₆-Halogenalkylsulfonyloxy, Phenylsulfonyloxy, p-Toluolsulfonyloxy, p-Chlorphenylsulfonyloxy, p-Bromphenylsulfonyloxy, oder p-Nitrophenylsulfonyloxy,
   insbesondere bevorzugt Chlor, Methylsulfonyloxy, Trifluormethylsulfonyloxy oder Phenylsulfonyloxy;
   wobei man eine Verbindung der Formel I erhält, worin die Variablen R², R³, X¹, X², X³, Ar und A die zuvor genannten Bedeutungen und vorzugsweise die bevorzugten Bedeutungen aufweisen und R¹ für Amino steht, oder mit
(B) einem Alkylierungsmittel der Formel V 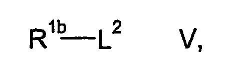
worin
- R^{1b}: für C₁-C₆-Alkyl, C₁-C₆-Halogenalkyl, C₃-C₇-Cycloalkyl, C₂-C₆-Alkenyl, C₂-C₆-Halogenalkenyl, C₃-C₆-Alkinyl oder C₃-C₆-Halogenalkinyl,
bevorzugt für C₁-C₆-Alkyl,
sehr bevorzugt für C₁-C₄-Alkyl; und
- L²: für eine nucleophil verdrängbare Abgangsgruppe,
bevorzugt für Halogen, Hydrogensulfat, C₁-C₆-Alkylsulfonyloxy, C₁-C₆-Halogenalkylsulfonyloxy oder Phenylsulfonyloxy,
wobei der Phenylring gegebenenfalls mit Halogen, Nitro, C₁-C₆-Alkyl oder C₁-C₆-Halogenalkyl ein- oder mehrfach substituiert ist,
besonders bevorzugt für Halogen, Hydrogensulfat, C₁-C₆-Alkylsulfonyloxy, C₁-C₆-Halogenalkylsulfonyloxy, Phenylsulfonyloxy, p-Toluolsulfonyloxy, p-Chlorphenylsulfonyloxy, p-Bromphenylsulfonyloxy, oder p-Nitrophenylsulfonyloxy, insbesondere bevorzugt Chlor, Methylsulfonyloxy, Trifluormethylsulfonyloxy oder Phenylsulfonyloxy,
steht;
wobei man eine Verbindung der allgemeinen Formel I erhält, worin die Variablen R², R³, X¹, X², X³, Ar und A die zuvor genannten Bedeutungen und vorzugsweise die bevorzugten Bedeutungen aufweisen und R¹ die für R^{1b} genannten Bedeutungen aufweist.

Das Verfahren zur Alkylierung oder Aminierung der Verbindung I mit R¹ ist insofern überraschend, da man die Bildung entsprechender N-Alkylsulfonamide oder Gemische aus N-Alkylsulfonamiden oder N-Alkyl substituierten (Thio)Uracilen bzw. Dithiouracilen erwartet hätte. Es ist bekannt, dass Schwefelsäurediamide in einfacher Weise mit Schwefelsäurediestern oder Arensulfonsäureestern in Gegenwart einer Base alkyliert werden, siehe beispielsweise R. Sowada, J. Prakt. Chem. 25, 88 (1964). Im Falle von trisubstituierten Schwefelsäurediamiden ist die Bildung von tetrasubstituierten Schwefelsäurediamiden bekannt, siehe B. Unterhalt, E. Seebach, Arch. Pharm. 314, 51 (1981). Ebenso lassen sich Schwefelsäurediamide, bei denen die Amidfunktion bereits einen Acylrest trägt, alkylieren, siehe K. C. C. Bancroft et al., J. Heterocycl. Chem. 15, 1521 (1978); A. Martinez et al., Bioorg. Med. Chem. Lett. 9 (21), 3133 (1999). Der Fachmann hätte daher - aufgrund der leichten Alkylierbarkeit der Sulfamindseitenkette - die bevorzugte Alkyierung am Sulfonamidstickktoffatom oder zumindest die Bild ung dialkylierter Produkte erwartet.

Die Einführung der Aminogruppe am (Thio)Uracilring beziehungsweise Dithiouracilring gelingt überraschenderweise in Anlehnung an bekannte Verfahren zur Einführung der Aminogruppe am Uracilstickstoff. Solche Verfahren sind beispielsweise in der DE 196 52431 beschrieben, auf deren Offenbarung zur elektrophilen Aminierung hiermit im vollen Umfang Bezug genommen. Als geeignete Aminierungsreagentien der Formel IV kommen beispielsweise 1-Aminooxy-2,4-dinitrobenzol oder O-Mesitylensulfonylhydroxylamin in Betracht.

Gegebenenfalls erfolgt die Umsetzung in Gegenwart einer Base. Als Base kommen alle üblichen anorganischen oder organischen Basen in Betracht. Geeignete Basen sind z.B. die im Zusammenhang mit der Herstellung der Verbindung I durch Umsetzung von II mit III genannten Basen. Bevorzugte Basen sind Alkalimetallalkoholate, insbesondere Lithium-, Natrium- oder Kaliumalkoholate wie Natriummethylat, Natriumethylat, Lithiumethylat, Kaliummethylat, Kaliumethylat, Kalium-tert.-butylat, Natrium-tert.-butylat, Natrium-isopropylat, Kalium-tert.-pentylat, Alkalimetallhydride wie Natriumhydrid, Kaliumhydrid, Alkalimetallcarbonate wie Lithiumcarbonat, Natriumcarbonat, Kaliumcarbonat, Cäsiumcarbonat oder tertiäre Amine, insbesondere Amidinbasen wie 1,8-Diazabicyclo[5.4.0]undec-7-en. In der Regel setzt man die Verbindung I mit R¹ = Wasserstoff und die Base annähernd äquimolar ein.

Die Umsetzung der Verbindung I mit R¹ = Wasserstoff mit einem Aminierungsreagenz der Formel IV erfolgt in der Regel in einem inerten organischen Lösungsmittel oder Lösungsmittelgemisch. Für diesen Zweck bevorzugte Lösungsmittel sind Nitrile wie Acetonitril, Propionitril oder Butyronitril, Ketone wie Aceton und Methylethylketon, Carbonate wie Dimethylcarbonat, Diethylcarbonat und Ethylencarbonat sowie Amide wie N,N-Dimethylformamid, N,N-Dimethylacetamid und N-Methylpyrrolidon. Geeignet sind auch organische Lösungsmittel mit basischem Charakter, z. B. die vorgenannten tertiären Amine wie Trialkylamine und Pyridinverbindungen.

In der Regel wird man die Umsetzung bei Temperaturen von 0 bis 80 °C, vorzugsweise zwischen 10 und 60 °C durchführen. Hierfür setzt man in der Regel die Verbindung I mit R¹ = Wasserstoff und das Aminierungsreagenz der Formel IV annähernd äquimolar ein. Es ist jedoch auch möglich, eine der Komponenten in einem größeren Überschuss zu verwenden, wobei der Überschuss vorzugsweise nicht mehr als 50 mol-%, bezogen auf die im Unterschuss vorliegende Komponente, betragen wird.

Die Aufarbeitung des erhaltenen Reaktionsgemisches erfolgt nach bekannten Verfahren, beispielsweise durch wässrig-extraktive Aufarbeitung. Auf diesem Weg können Phenyl(thio)uracile und Phenyldithiouracile I mit R¹ = NH₂ auf einfachem und wirtschaftlichem Weg hergestellt werden.

In einer weiteren Variante des erfindungsgemäßen Verfahrens setzt man zunächst das Enamin der Formel III mit R^{1a} = Wasserstoff in Gegenwart eines Überschusses an Base mit dem Phenyliso(thio)cyanat der Formel II um, ohne die Verbindung I mit R¹ = Wasserstoff zu isolieren oder zu reinigen. Danach versetzt man dann das Reaktionsgemisch mit einem Aminierungsmittel der allgemeinen Formel IV, so dass man direkt die Verbindung I mit R¹ = Amino erhält.

Die N-Alkylierung der Verbindung I am freien (Thio)Uracilstickstoffatom gelingt in für Uracile an sich bekannter Weise durch Umsetzung der Verbindung I mit R¹ = Wasserstoff mit einem Alkylierungsmittel R^{1b}-L² (V), wie sie beispielsweise in der US 4,943,309 beschrieben sind, auf deren Offenbarung zur Alkylierung hiermit im vollen Umfang Bezug genommen.

Beispiele für eine geeignete, nucleophil verdrängbare Abgangsgruppe L² sind Halogenid, vorzugsweise Chlorid, Bromid oder Iodid, Sulfat, Phenylsulfonyloxy, worin der Phenylrest gegebenenfalls mit Halogen, Nitro oder C₁-C₆-Alkyl ein- oder mehrfach, substituiert ist wie Phenylsulfonyloxy, p-Toluolsulfonyloxy, p-Chlorphenylsulfonyloxy, p-Bromphenylsulfonyloxy oder p-Nitrophenylsulfonyloxy, C₁-C₆-Alkysulfonyloxy wie Methylsulfonyloxy, C₁-C₆-Halogenalkylsulfonyloxy wie Trifluormethylsulfonyloxy.

R^{1b} steht vorzugsweise für C₁-C₄-Alkyl.

Bevorzugte Alkylierungsmittel sind somit C₁-C₄-Alkylhalogenide, Di-C₁-C₄-alkylsulfate, Phenylsulfonsäure-C₁-C₄-alkylester, wobei der Phenylrest gegebenenfalls mit Halogen, Nitro oder C₁-C₆-Alkyl ein- oder zweifach substituiert ist. Besonders bevorzugte Alkylierungsmittel sind Methylierungsmittel oder Ethylierungsmittel wie Dimethylsulfat, Diethylsulfat, Methyliodid, Ethyliodid, Methylbromid, Methylchlorid, Ethylbromid, Ethylchlorid, C₁-C₆-Alkylsulfonsäuremethylester oder -ethylester oder die Methyl- oder Ethylester der zuvor genannten Phenylsulfonsäuren. Ein ganz besonders bevorzugtes Methylierungsmittel ist Dimethylsulfat.

Im erfindungsgemäßen Verfahren kann man das Alkylierungsmittel sowohl in äquimolarer Menge, bezogen auf die Verbindung I, als auch in substöchiometrischer Menge oder überstöchiometrischer Menge einsetzen. Üblicherweise setzt man wenigstens eine äquimolare Menge an Alkylierungsmittel V, bezogen auf die Verbindung I ein. Die molaren Verhältnisse, in denen die Verbindung I mit R¹ = Wasserstoff zu Alkylierungsmittel V eingesetzt werden, liegen in der Regel im Bereich von 1:1 bis 1:3, vorzugsweise 1:1 bis 1:1,3 für das Verhältnis von Verbindung I zu Alkylierungsmittel V.

Üblicherweise führt man die Alkylierung in Gegenwart einer Base durch. Als Base kommen grundsätzlich alle Verbindungen in Betracht, die in der Lage sind, das Lactamstickstoffatom zu deprotonieren Geeignete Basen sind z.B. die im Zusammenhang mit der Herstellung der Verbindung I durch Umsetzung von II mit III genannten Basen. Bevorzugt ist die Base ausgewählt unter Alkali- und Erdalkalimetallhydroxiden wie Natriumhydroxid, Kaliumhydroxid und Lithiumhydroxid, Alkali- und Erdalkalimetalloxiden wie Calciumoxid, Alkali- und Erdalkalimetallcarbonaten wie Lithiumcarbonat, Natriumcarbonat, Kaliumcarbonat, Cäsiumcarbonat, Magnesiumcarbonat, Calciumcarbonat, Zinkcarbonat oder Bariumcarbonat. In einer besonders bevorzugten Ausführungsform des erfindungsgemäßen Verfahrens setzt man als Base Natriumhydroxid oder Kaliumcarbonat ein.

Die Base kann in substöchiometrischer, überstöchiometrischer oder äquimolarer Menge, bezogen auf die Verbindung I, eingesetzt werden. Vorzugsweise setzt man wenigstens eine äquimolare Menge an Base, bezogen auf die Verbindung I ein. Die Menge an Base wird in der Regel nicht mehr als 1,3 mol, bezogen auf 1 mol der Verbindung I, betragen.

Die Umsetzung der Verbindungen I mit R¹ = Wasserstoff mit dem Alkylierungsmittel der Formel V wird vorteilhaft in Gegenwart eines Lösungsmittels durchgeführt. Als Lösungsmittel für diese Umsetzungen verwendet man - je nach Temperaturbereich - aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offfenkettige Dialkylether wie Diethylether, Di-n-proplyether, Di-n-isopropylether, Methyl-tert-butylether, cyclische Ether wieTetrahydrofuran, 1,4-Dioxan, Anisol, Glykolether wie Dimethylglykolether, Diethylglykolether, Diethylenglykoldimethylether, Diethylenglykoldiethylether, C₁-C₄-Alkohole wie Methanol, Ethanol, n-Propanol, iso-Propanol, n-Butanol, Ketone wie Aceton, Methylethylketon, Methylisopropylketon, Methylisobutylketon, Butanon, Carbonate wie Diethylcarbonat und Ethylencarbonat, N,N-Dialkylamide wie N,N-Dimethylformamid oder N,N-Dimethylacetamid, N-Alkyllactame wie N-Methylpyrrolidon, Sulfoxide wie Dimethylsulfoxid, Tetraalkylharnstoffe wie Tetramethylharnstoff, Tetraethylharnstoff, Tetrabutylharnstoffe, Dimethylethylenharnstoff, Dimethylpropylenharnstoff oder Gemische dieser Lösungsmittel. Als Lösungsmittel bevorzugt sind N,N-Dimethylformamid, N-Methylpyrrolidon, Aceton, Dichlormethan, Tetrahydrofuran, Toluol oder Gemische dieser Lösungsmittel.

Vorzugsweise führt man die Alkylierung der Verbindung I bei Temperaturen zwischen -5 °C und 100 °C, vorzugsweise bei Temperaturen zwischen 0 °C und 80 °C und insbesondere bei Temperaturen zwischen 20 °C und 50 °C durch. Die Reaktionszeit kann der Fachmann in an sich üblicher Weise durch Routinemethoden wie Dünnschichtchromatographie oder HPLC ermitteln.

Die Zugabe von Verbindung I, Alkylierungsmittel V und Base kann getrennt, gleichzeitig oder nacheinander erfolgen.

Vorteilhaft kann man das mehrstufige Verfahren zur Herstellung der Verbindung I mit R¹ ≠ Wasserstoff auch als Eintopfreaktion durchführen. Bei der Umsetzung des Phenyliso(thio)cyanats der Formel II mit dem Enamin der Formel III mit R^{1a} = Wasserstoff in Gegenwart eines Überschusses an Base entsteht zunächst das Uracilsalz, das ohne Isolierung oder Reinigung anschließend mit dem Alkylierungsmittel umgesetzt wird. Danach führt man die Reaktion im angegebenen Temperaturbereich zu Ende.

In einer anderen Variante des erfindungsgemäßen Verfahrens kann man die Umsetzung auch in einem wässrigen Mehrphasensystem durchführen, vorzugsweise in Gegenwart von Phasentransferkatalysatoren wie quartären Ammoniumsalzen oder Phosphoniumsalzen. Geeignete quartäre Ammoniumsalze umfassen Tetraalkyl-(C₁-C₁₈)-ammoniumchloride, -bromide, fluoride oder tetrafluoroborate wie Tetraethylammoniumchlorid, Tetrabutylammoniumbromid, Tetrabutylammoniumiodid, Tetrabutylammoniumtetrafluoroborat, N-Benzyltrialkyl-(C₁-C₁₈)-ammoniumchloride, -bromide oder -fluoride wie Benzyltriethylammoniumchlorid, vorzugsweise Tetrabutylammoniumbromid oder Tetrabutylammoniumiodid. Geeignete Phosphoniumsalze sind beispielsweise Tetraphenylphosphoniumchlorid oder -bromid, Tetraalkyl-(C₁-C₁₈)-phosphoniumchlorid oder -bromid wie Tetrabutylphosphoniumbromid. In der Regel setzt man den Phasentransferkatalysator in einer Menge von bis zu 20 mol-%, vorzugsweise zwischen 1 und 15 mol-% und insbesondere zwischen 2 und 12 mol.-%, bezogen auf die Verbindung I mit R¹ = Wasserstoff, ein.

Das Mehrphasensystem umfasst eine wässrige Phase und wenigstens eine organische flüssige Phase. Daneben können im Verlauf der Umsetzung auch feste Phasen auftreten. Die wässrige Phase ist vorzugsweise eine Lösung von Alkali- oder Erdalkalihydroxiden oder -carbonaten in Wasser. Bezüglich geeigneter Alkali- oder Erdalkalihydroxide oder -carbonate wird auf das zuvor Gesagte verwiesen. Besonders bevorzugt verwendet man Alkali- oder Erdalkalihydroxide, speziell Natriumhydroxid. Vorzugsweise kommen für die organische Phase aliphatische, cycloaliphatische oder aromatische, gegebenenfalls halogenierte Kohlenwasserstoffe, cyclische oder offenkettige Ether oder Gemische davon in Betracht, wobei bezüglich der aliphatischen, cycloaliphatischen oder aromatischen, gegebenenfalls halogenierten Kohlenwasserstoffe, cyclischen oder offenkettigen Ether auf das zuvor Gesagte Bezug genommen wird. In einer bevorzugten Ausführungsform des erfindungsgemäßen Verfahrens besteht das Mehrphasensystem aus wässriger Natriumhydroxid-Lösung als wässrige Phase und aus Toluol und Tetrahydrofuran oder Dichlormethan und Tetrahydrofuran als organische Phase.

Bei Verwendung eines Mehrphasensystems kann man beispielsweise die Verbindung I in einem der vorgenannten organischen Lösungsmittel oder Lösungsmittelgemische vorlegen. Danach gibt man die wässrige Lösung der Base, das Alkylierungsmittel und den Phasentransferkatalysator unter Durchmischen zu und bringt dann im genannten Temperaturbereich die Umsetzung zu Ende.

Die Umsetzung kann bei Normaldruck, vermindertem Druck oder unter erhöhtem Druck, gegebenenfalls unter Inertgas kontinuierlich oder diskontinuierlich durchgeführt werden.

Die Aufarbeitung des Reaktionsgemisches zur Gewinnung des Zielproduktes I kann nach den hierfür üblichen Methoden erfolgen. In der Regel wird man das verwendete Lösungsmittel nach üblichen Verfahren, beispielsweise destillativ, entfernen. Man kann dann die Zielverbindung I in einem mit Wasser nicht mischbaren organischen Lösungsmittel aufnehmen, extrahiert etwaige Verunreinigungen mit gegebenenfalls angesäuertem Wasser, trocknet und entfernt das Lösungsmittel unter vermindertem Druck. Zur weiteren Reinigung kann man die üblichen Verfahren wie Kristallisation, Fällung oder Chromatographie anwenden. Bei Verwendung eines Zweiphasensystems wird man in der Regel extraktiv aufarbeiten.

Verbindungen der Formel I, worin einer der Reste X¹, X² oder X³ oder die Reste X¹, X² und X³ jeweils für Sauerstoff stehen, können nach bekannten Methoden durch Behandeln mit Schwefelungsmitteln in Verbindungen der allgemeinen Formel I umgewandelt werden, worin einer der Reste X¹, X² oder X³ oder die Reste X¹, X² und X³ jeweils für Schwefel stehen. Beispiele für geeignete Schwefelungsmitteln sind Organophosphorsulfide wie das Lawesson-Reagenz, Organozinnsulfide oder Phosphor(V)sulfide (siehe auch J. March, Advanced Organic Synthesis, 2. Auflage, Wiley Interscience 1985, S. 794 und dort zitierte Literatur). Die Umsetzung kann in einem Lösungsmittel oder in Substanz durchgeführt werden. Geeignete Lösungsmittel sind die oben genannten, inerten Lösungsmittel sowie basische Lösungsmittel wie Pyridin und vergleichbare. Die zur Umsetzung erforderliche Temperatur liegt in der Regel oberhalb Raumtemperatur und liegt insbesondere im Bereich von 50 bis 200 °C. Führt man die Umsetzung des Enamins IIII mit einem Isothiocyanat II, in dem der Rest X¹ für Schwefel steht durch, so erhält man direkt die entsprechenden 2-Thioxouracile mit X¹ = Schwefel.

Das erfindungsgemäße Verfahren liefert die Uracilverbindungen der Formel I in guten Gesamtausbeuten und mit hoher Reinheit. Zudem ist es weniger aufwendig als die Verfahren des Standes der Technik.

Die folgenden Beispiele dienen der Erläuterung der Erfindung.

### Beispiel 1: Herstellung von 2-Chlor-5-[3,6-dihydro-2,6-dioxo-4-(trifluormethyl)-1(2H)-pyrimidinyl]-4-fluor-N-[[methyl(1-methylethyl)amino]sulfonyl]-benzamid unter Verwendung von Natriumhydrid als Base

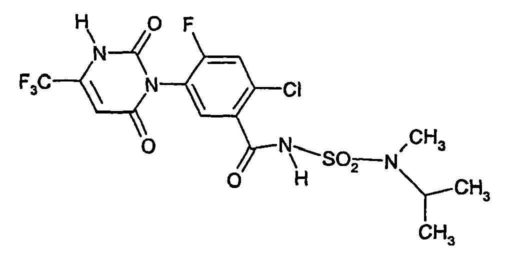

Unter Stickstoff rührte man ein Gemisch aus 1,70 g (9,29 mmol) 3-Amino-4,4,4-trifluorcrotonsäureethylester in 20 ml N,N-Dimethylformamid und 20 ml n-Hexan 40 Minuten am Wasserabscheider unter Rückfluss. Danach entfernte man das n-Hexan unter vermindertem Druck, kühlte das verbliebene Gemisch auf 5 bis 8 °C und gab unter Rühren in 5 Portionen 0,8 g (20 mmol) 60%iges Natriumhydrid (in Mineralöl) zu. Nach 15minütigem Nachrühren gab man zu der gelblichen Lösung unter Rühren eine Lösung von 2,8 g (8,0 mmol) N-(2-Chlor-4-fluor-5-isocyanatobenzoyl)-N'-methyl-(1-methylethyl)sulfamid in 10 ml Tetrahydrofuran und rührte 2 Stunden, zuletzt bei 22 °C nach.

Man versetzte das Reaktionsgemisch unter Rühren mit 2,0 g (33 mmol) Eisessig und 80 ml Wasser. Nach 40minütigem Rühren setzte Kristallisation ein. Zur Vervollständigung der Kristallisation stellte man den pH-Wert des wässrigen Reaktionsgemisches mit konz. Salzsäure auf pH 2 und gab weitere 40 ml Wasser zu. Man saugte den entstandenen fein kristallinen, leicht gelblichen Niederschlag ab und wusch mit Wasser und Hexan. Nach dem Trocknen in Methylenchlorid über Natriumsulfat engte man das Lösungsmittel im Vakuum bis zur Trockne ein, wobei man 3,9 g (100 % der Theorie) der Titelverbindung mit einem Schmelzpunkt von 233 - 236 °C (Zersetzung) erhielt. ¹H-NMR (400 MHz, DMSO-d₆) δ (ppm): 12,8 (br, NH), 12,25 (s, NH), 7,82 (d, 1H), 7,76 (d, 1H), 6,4 (s, 1H), 4,1 (m, 1H), 2,8 (s, 3H), 1,12 (d, 3H), 1,12 (d, 6H).

### Beispiel 2: Herstellung von 2-Chlor-5-[3,6-dihydro-2,6-dioxo-4-(trifluormethyl)-1(2H)-pyrimidinyl]-4-fluor-N-[[methyl(1-methylethyl)amino]sulfonyl]-benzamid unter Verwendung von Kaliummethoxid als Base

Auf die im Beispiel 1 beschriebene Weise behandelte man 1,70 g (9,29 mmol) 3-Amino-4,4,4-trifluorcrotonsäureethylester in 20 ml N,N-Dimethylformamid mit n-Hexan. Danach kühlte man das verbliebene Gemisch auf -12 °C und gab in einer Portion 1,47 g (19,9 mmol) 95%iges Kaliummethoxid unter Rühren zu. Man rührte das Gemisch 15 Minuten bei -15 °C. Zu der gelblichen Lösung gab man innerhalb 10 Minuten unter Rühren bei -10 °C bis -15°C eine Lösung von 2,8 g (8,0 mmol) N-(2-Chlor-4-fluor-5-isocyanatobenzoyl)-N'-methyl-(1-methylethyl)sulfamid in 10 ml Tetrahydrofuran und rührte 3 Stunden bei -10 °C bis -12 °C. Man untersuchte das Reaktionsgemisch dünnschichtchromatographisch und stellte fest, dass während der letzten beiden Stunden keine Änderung der Reaktionszusammensetzung erfolgt war.

Zur Aufarbeitung des Reaktionsgemisches versetzte man mit 2,0 g (33 mmol) Eisessig und 120 ml Wasser, stellte das wässrige Reaktionsgemisch mit konz. Salzsäure auf pH 2 und saugte den ausgefallenen Niederschlag ab. Zur rascheren Trocknung löste man den feuchten Niederschlag in Dichlormethan unter Zusatz von 5 Gew.-% Methanol, wusch mit einer gesättigten Kochsalzlösung und trennte die organische Phase ab. Nach dem Trocknen über Natriumsulfat und Einengen im Vakuum erhielt man 3,16 g (81 % der Theorie) der Titelverbindung mit einem Schmelzpunkt von 230 - 233 °C (Zersetzung). Laut HPLC-Analyse war die Verbindung zu 98,2 % rein (HPLC-Säule: 250 x 4 mm, RP 18 LiChrospher, 100 (5 µm) Fa. Merck, mobile Phase: Acetonitril/Wasser 60/40 Vol.-% über 1 Minute, danach 80/20 Vol.-% über 7 Minuten und abschließend 60/40 Vol.%; Flussrate: 1 ml/min, UV 254 nm, RT: 1,26 Minuten.

### Beispiel 3: Herstellung von 2-Chlor-5-[3,6-dihydro-2,6-dioxo-4-(trifluormethyl)-1(2H)-pyrimidinyl]-4-fluor-N-[[methyl(1-methylethyl)amino]sulfonyl]-benzamid Verfahren unter Verwendung von Kaliumcarbonat als Base

Unter Stickstoff erhitzte man ein Gemisch aus 3,3 g (23,8 mmol) Kaliumcarbonat in 20 ml N,N-Dimethylformamid und 25 ml n-Hexan unter Rühren 30 Minuten am Wasserabscheider bei einer Innentemperatur von 70 °C am Rückfluss. Man ließ das Gemisch unter Stickstoff auf 40 °C abkühlen und gab danach 1,7 g (9,29 mmol) 3-Amino-4,4,4-trifluorcrotonsäureethylester zu, erhitzte weitere 30 Minuten am Rückfluss und entfernte danach das n-Hexan unter vermindertem Druck. Unter Rühren gab man zu dem erhaltenen, auf 22 °C abgekühlten, leicht rötlichen Gemisch 2,8 g (8,0 mmol) N-(2-Chlor-4-fluor-5-isocyanatobenzoyl)-N'-methyl-(1-methylethyl)sulfamid in 10 ml Tetrahydrofuran und rührte 30 Minuten bei 22 °C und danach 90 Minuten bei 50 bis 55 °C. Man untersuchte das Reaktionsgemisch mit HPLC unter den in Beispiel 2 beschriebenen Bedingungen und stellte fest, dass das Reaktionsgemisch einer im Vakuum eingeengten Probe 45 % der Theorie der Titelverbindung mit RT = 1,26 Minuten enthielt.

### Beispiel 4: Herstellung von 2-Chlor-5-[3,6-dihydro-3-methyl-2,6-dioxo-4-(trifluormethyl)-1(2H)-pyrimidinyl]-4-fluor-N-[[methyl-(1-methylethyl)amino]sulfonyl]-benzamid durch Umsetzung von N-(2-Chlor-4-fluor-5-isocyanatobenzoyl)-N'-methyl-(1-methylethyl)sulfamid mit 3-Methylamino-4,4,4-trifluorcrotonsäureethylester

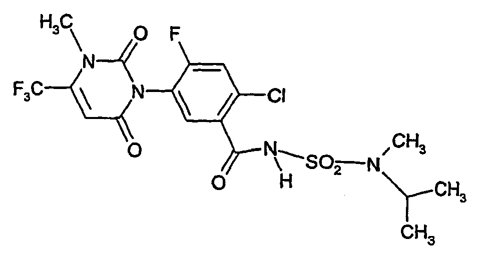

Unter Stickstoff rührte man 0,99 g (5,021 mmol) 3-Methylamino-4,4,4-trifluorcrotonsäureethylester in 25 ml N,N-Dimethylformamid und 50 ml n-Pentan 45 Minuten am Wasserabscheider am Rückfluss. Anschließend destillierte man das n-Pentan bis zur einer Innentemperatur von 70 °C ab. Man ließ auf 40 °C abkühlen und gab danach innerhalb 15 Minuten bei einer Temperatur von bis zu 45 °C 1,13 g (10,043 mmol) Kalium-tert-butylat in 3 Portionen unter Rühren zu, wobei eine rotbraune Lösung entstand. Nach 20minütigem Rühren bei 40 °C ließ man abkühlen und gab dann bei -15 °C bis - 10 °C 1,55 g (4,419 mmol) N-(2-Chlor-4-fluor-5-isocyanatobenzoyl)-N'-methyl-(1-methylethyl)sulfamid innerhalb 2 Minuten zu, wobei sofortige Lösung eintrat. Man rührte 30 Minuten bei -10 °C, ließ danach das Reaktionsgemisch auf 22 °C erwärmen und rührte noch 30 Minuten bei dieser Temperatur nach.

Unter leichtem Kühlen säuerte man bei 20 - 22 °C das erhaltene Reaktionsgemisch mit 0,46 g (12,553 mmmol) 4 n Salzsäure in 3,1 ml Dioxan an und engte im Vakuum ein. Den erhaltenen Rückstand verteilte man in einem Lösungsmittelgemisch aus 100 ml Methyl-tert.-butylether und 100 ml Wasser. Die organische Phase trennte man ab, trocknete über Magnesiumsulfat, filtrierte über eine Alltech-Fertigsäule (10 g/60 ml) und engte danach im Vakuum bis zur Trockne ein. Den erhaltenen Rückstand rührte man in Methyl-tert.-butylether 0,5 h bei 0 °C, saugte ab, wusch mit Methyl-tert.-butylether und trocknete im Vakuum, wobei man 0,97 g (41,6 % der Theorie) der Titelverbindung als glasartiges Harz mit einer ¹H-NMR-Reinheit von 95 % erhielt. Aus dem Filtrat erhielt man beim Einengen 0,9 g eines glasartigen Harzes, das nach dem ¹H-NMR-Spektrum noch etwa 0,45 g (20,3 % der Theorie) der Titelverbindung enthielt.
¹H-NMR (400 MHz, CDCl₃) δ (ppm): 9,5 (br, NH), 7,63 (d, 1 H), 7,37 (d, 1 H), 6,37 (s, 1H), 4,29 (m, 1 H), 3,58 (s, 3H), 2,92 (s, 3H), 1,18 (d, 6H).

### Beispiel 5: Herstellung von 2-Chlor-5-[3,6-dihydro-3-methyl-2,6-dioxo-4-(trifluormethyl) 1(2H)-pyrimidinyl]-4-fluor-N-[[methyl-(1-methylethyl)aminolsulfonyl]-benzamid durch Methylierung von 2-Chlor-5-[3,6-dihydro-2,6-dioxo-4-(trifluormethyl)-1(2H)-pyrimidinyl]-4-fluor-N-[[methyl(1-methylethyl)amino]suflonyl]-benzamid - ohne Phasentransferkatalyse

Zu 2,0 g (4,11 mmol) 2-Chlor-5-[3,6-dihydro-2,6-dioxo-4-(trifluoromethyl)-1(2H)-pyrimidinyl]-4-fluor-N-[[methyl(1-methylethyl)amino]sulfonyl]benzamid aus Beispiel 1 in 80 ml N,N-Dimethylformamid gab man 1,14 g (9,04 mmol) Dimethylsulfat und 0,283 g (2,055 mmol) K₂CO₃ und rührte danach 16 Stunden bei 25°C. Anschließend destillierte man bei 30 °C und reduziertem Druck das N,N-Dimethylformamid ab und nahm den Rückstand in etwa 250 ml Ethylacetat auf. Man säuerte das Reaktionsgemisch mit 10 %iger HCl an und extrahierte danach zweimal mit Wasser. Man trocknete die organische Phase über MgSO₄ und destillierte das Lösungsmittel ab, wobei man 1,95 g des rohen Produktes erhielt. Die Reinheit des Wertproduktes betrug laut ¹H-NMR und HPLC 77 % (entspricht einer Ausbeute von 73%). Zur Reinigung chromatographierte man 0,92 g dieses rohen Produktes an Kieselgel (Säule 28 x 4,5 cm) mit Cyclohexan/Essigester 9/1 bis 1/1, wobei man vier Fraktionen erhielt. Die 3. Fraktion (0,58 g; entspricht 59% isolierter Ausbeute) enthielt das gewünschte Wertprodukt in reiner Form.
¹H-NMR-Daten (DMSO-d₆) δ(ppm) : 12,2 (NH), 7,8 (d, 1 H), 7,7 (d, 1 H), 6,6 (s, 1 H), 4,1 (sept, 1 H), 3,5 (s, 3 H), 3,3 (s, 3 H), 2,9 (s, 3 H), 1,2 (d, 6 H)

### Beispiel 6: Herstellung von 2-Chlor-5-[3,6-dihydro-3-methyl-2,6-dioxo-4-(trifluormethyl)-1(2H)-pyrimidinyl]-4-fluor-N-[[methyl-(1-methylethyl)aminolsulfonyl]-benzamid durch Methylierung von 2-Chlor-5-[3,6-dihydro-2,6-dioxo-4-(trifluormethyl)-1(2H)-pyrimidinyl]-4-fluor-N-[[methyl(1-methylethyl)amino]suflonyl]-benzamid - unter Phasentransferkatalyse (mit Tetrahydrofuran und Toluol als organische Phase und Tetrabutylammoniumbromid als Phasentransferkatalysator)

Zu einem Lösungsmittelgemisch aus 135 g Toluol und 27 g Tetrahydrofuran gab man bei 25 °C 12,45 g (0.024 mol) 2-Chlor-5-[3,6-dihydro-2,6-dioxo-4-(trifluormethyl)-1(2H)-pyrimidinyl]-4-fluor-N-[[methyl(1-methylethyl)amino]sulfonyl]benzamid (93,9%ig) aus Beispiel 1 und versetzte danach das Gemisch mit einer Lösung aus 2,3 g (0,0288 mol) Natriumhydroxid (50%ig) in 57,5 g Wasser. Zu dem Reaktionsgemisch gab man 0,77 g (0,0024 mol) Tetrabutylammoniumbromid und 3,69 g (0,0293 mol) Dimethylsulfat zu. Das zweiphasige Reaktionsgemisch rührte man 23 Stunden intensiv bei 25°C.

Danach trennte man die wässrige Phase ab und wusch die organische Phase zweimal mit jeweils 100 ml Wasser. Nach dem Trocknen der vereinten organischen Phase destillierte man das Lösungsmittel bei reduziertem Druck ab, wobei man 13,8 g eines rohen Produktes erhielt, welches nach quantitativer HPLC die Titelverbindung zu 77,5% enthielt (entspricht einer Ausbeute von 88,9%).

### Beispiel 7: Herstellung von 2-Chlor-5-[3,6-dihydro-3-methyl-2,6-dioxo-4-(trifluormethyl)-1(2Hl-pyrimidinyl]-4-fluor-N-[[methyl-(1-methylethyl)amino]sulfonyl]-benzamid durch Methylierung von 2-Chlor-5-[3,6-dihydro-2,6-dioxo-4-(trifluorrnethyl)-1(2H)-pyrimidinyl]-4-fluor-N-[[methyl(1-methylethyl)amino]suflonyl]-benzamid - unter Phasentransferkatalyse (unter Verwendung von Tetrahydrofuran und Methylenchlorid als organische Phase und Tetrabutylammoniumiodid als Phasentransferkatalysator)

Zu einem Lösungsmittelgemisch aus 250 ml Dichlormethan und 125 ml Tetrahydrofuran gab man 5 g (10,3 mmol) 2-Chlor-5-[3,6-dihydro-2,6-dioxo-4-(trifluoromethyl)-1 (2H)-pyrimidinyl]-4-fluor-N-[[methyl(1-methylethyl)amino]sulfonyl]benzamid aus Beispiel 1 und versetzte danach mit einer Lösung von 0,411 g (10,3 mmol) NaOH in 375 ml Wasser. Zu dem Reaktionsgemisch gab man 0,38 g (1,03 mmol) Tetrabutylammoniumiodid und 1,36 g (10,8 mmol) Dimethylsulfat und rührte das Zwei-Phasen-Gemisch 14 Stunden mit 1000 Umdrehungen/min.

Man trennte die wässrige Phase ab und engte die organische Phase unter vermindertem Druck bis zur Trockne ein. Die chromatographische Reinigung an Kieselgel erfolgte auf die in Beispiel 5 beschriebene Weise, wobei man 4 Fraktionen erhielt. Nach Entfernen des Lösungsmittels enthielt die erste Fraktion 0,54 g eines Gemisches, das gemäß ¹H-MNR zu 90 % aus dem gewünschten Wertprodukt bestand und die zweite Fraktion 2 enthielt 2,4 g des Wertprodukts mit einer Reinheit > 95 % (Ausbeute bezogen auf beide Fraktionen: 56%).

### Beispiel 8: 2-Chlor-5-[3,6-dihydro-3-methyl-4-(trifluormethyl)-6-oxo-2-thioxo-1-(2H)-pyrimidinyl]-4-fluor N-[[(1-methylethyl)-propyl-amino]sulfonyl]-benzamid

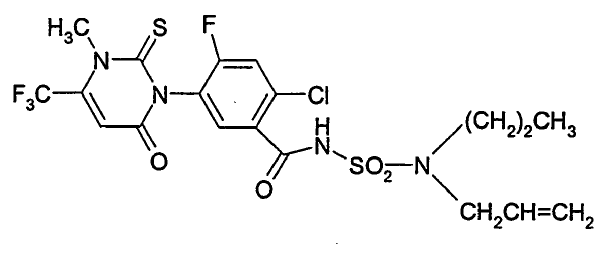

Unter Stickstoff erhitzte man ein Gemisch aus 30 ml N,N-Dimethylformamid und 50 ml n-Hexan unter Rühren 1 Stunde am Rückfluss und destillierte anschließend das Hexan bei einer Innentemperatur von 80 - 90 °C ab. Man ließ auf 30 °C abkühlen und gab unter Rühren 0,75 g (3,828 mmol) 3-Methylamino-4,4,4-trifluorcrotonsäureethylester zu, kühlte das Reaktionsgemisch auf -20 °C und gab in 3 Portionen 0,2 g (7,92 mmol) 95%iges Natriumhydrid unter Rühren zu, wobei sich ein gelber Niederschlag bildete. Man rührte noch 15 Minuten bei -15 °C nach und gab danach bei -15 °C zu dem Gemisch 1,5 g (3,828 mmol) N-(2-Chlor-4-fluor-5-isothiocyanatobenzoyl)-N'-allyl-(1-propyl)sulfamid. Nach 15minütigem Rühren bildete sich eine braune Lösung. Man rührte noch 1 Stunde bei -15 °C und danach 8 Stunden bei 22 °C nach. Das Reaktionsgemisch goss man unter Rühren in 100 ml 1 N Salzsäure und extrahierte das wässrige Gemisch dreimal mit Methyl-tert.-butylether. Die vereinigten organischen Phasen extrahierte man erneut mit 1 n Salzsäure, wusch danach die organische Phase mit Wasser und trocknete die organische Phase über Magnesiumsulfat. Nach dem Abfiltrieren des Trockenmittels engte man das Filtrat im Vakuum ein. Den Rückstand reinigte man durch Flash-Chromatographie an Kieselgel (3 x 20 cm Säule, Eluierungsmittel: Dichlormethan) und erhielt nach dem Einengen des Eluats im Vakuum 0,65 g (31,3 % der Theorie) der Titelverbindung mit einem Schmelzpunkt von 74 - 75 °C. Nach dem ¹H-NMR-Spektrum lag ein Rotamerengemisch im Verhältnis von 7:3 vor. Laut HPLC-Analyse lagen hierfür die Produktpeaks bei 5,3 und 5,48 Minuten mit 70 beziehungsweise 25 Flächenprozent.

### Beispiel 9: 2-Chlor-5-[3,6-dihydro-3-methyl-4-(trifluormethyl)-6-oxo-2-thioxo-1-(2H)-pyrimidin]-4-fluor N-[[(methylethyl)l-propargylamino]sulfonyl]-benzamid]

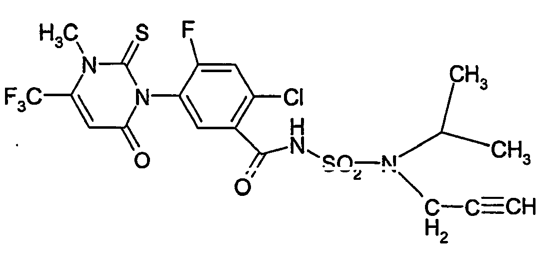

Auf die in Beispiel 8 beschriebene Weise erhielt man ausgehend von 1,0 g N-(2-Chlor-4-fluor-5-isothiocyanatobenzoyl)-N'-propargyl-(1-methylethyl)sulfamid und 0,61 g (3,078 mmol) 3-Methylamino-4,4,4-trifluorcrotonsäureethylester 0,388 g (28 % der Theorie) der Titelverbindung als 6:4 Rotamerengemisch mit einem Schmelzpunkt von 94 -105 °C.

## Patentansprüche

1. Verfahren zur Herstellung von 3-Phenyl(thio)uracilen oder 3-Phenyldithiouracilen der Formel I 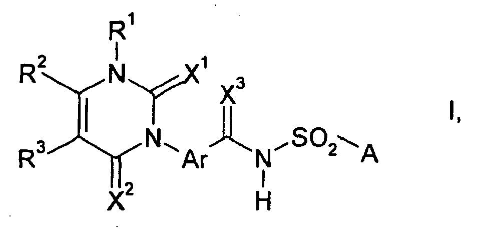 worin die Variablen die folgenden Bedeutungen haben:
R¹ Wasserstoff, Cyano, Amino, C₁-C₆-Alkyl, C₁-C₃-Cyanoalkyl, C₁-C₆-Halogenalkyl, C₁-C₆-Halogenalkoxy, C₃-C₇-Cycloalkyl, C₂-C₆-Alkenyl, C₂-C₆-Halogenalkenyl, C₃-C₆-Alkinyl, C₃-C₆-Halogenalkinyl oder Phenyl-C₁-C₄-alkyl;
R² und R³ unabhängig voneinander
Wasserstoff, C₁-C₆-Alkyl, C₁-C₆-Halogenalkyl, C₃-C₇-Cycloalkyl, C₂-C₆-Alkenyl, C₂-C₆-Halogenalkenyl, C₃-C₆-Alkinyl oder C₃-C₆-Halogenalkinyl;
X¹, X² und X³ unabhängig voneinander Sauerstoff oder Schwefel;
Ar Phenyl, das durch folgende Gruppen ein- oder mehrfach substituiert sein kann: Wasserstoff, Halogen, Cyano, C₁-C₄-Alkyl oder C₁-C₄-Halogenalkyl; und
A ein von einem primären oder sekundären Amin abgeleiteter Rest oder NH₂;
umfassend die Umsetzung eines Phenyliso(thio)cyanats der Formel II 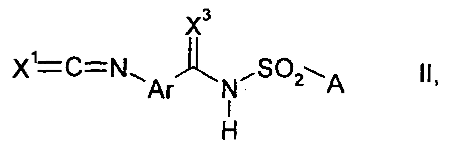 worin die Variablen X¹, X³, Ar und A die zuvor genannten Bedeutungen aufweisen,
mit einem Enamin der Formel III 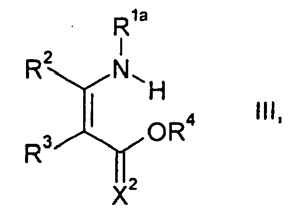 worin
R^{1a} die zuvor für R¹ genannten Bedeutungen mit Ausnahme von Amino aufweist;
R², R³ und X² die zuvor genannten Bedeutungen aufweisen; und
R⁴ für C₁-C₆-Alkyl, C₁-C₆-Halogenalkyl, C₁-C₃-Alkoxy-C₁-C₃-alkyl, C₁-C₃-Alkthio-C₁-C₃-alkyl, C₂-C₆-Alkenyl, C₂-C₆-Halogenalkenyl, C₃-C₆-Alkinyl, C₃-C₆-Halogenalkinyl, C₃-C₇-Cycloalkyl, C₁-C₆-Cyanoalkyl oder Benzyl, das seinerseits unsubstituiert oder am Phenylring durch Methyl, Methoxy, Methylthio, Halogen, Nitro oder Cyano substituiert ist, steht;
in Gegenwart von 1,8 bis 2,6 Äquivalenten Base pro Mol des Phenyliso(thio)-cyanates der Formel II;
und gegebenenfalls in einem weiteren Schritt die Umsetzung des erhaltenen 3-Phenyl(thio)uracils oder 3-Phenyldithiouracils der Formel I mit R¹=R^{1a}, wenn R¹ für Wasserstoff steht, mit einem Aminierungsmittel der Formel IV
H₂N-L¹ IV,
wobei L¹ für eine nucleophil verdrängbare Abgangsgruppe steht,
zu 3-Phenyl(thio)uracilen oder 3-Phenyldithiouracilen der Formel I mit R¹=Amino.

2. Verfahren nach Anspruch 1, **dadurch gekennzeichnet, dass** die Umsetzung in Gegenwart einer Base erfolgt, die ausgewählt ist unter Alkali- und Erdalkalicarbonaten, Alkali- und Erdalkalimetallalkoholaten, Alkali- und Erdalkalihydriden und tertiären Aminen.

3. Verfahren nach einem der vorherigen Ansprüche, **dadurch gekennzeichnet, dass** die Umsetzung in wenigstens einem aprotischen, polaren Lösungsmittel erfolgt, und das aprotische, polare Lösungsmittel einen Wassergehalt von 0 bis 0,5 Gew.-% , bezogen auf die Gesamtmenge an Verbindung II, Verbindung III und Lösungsmittel, aufweist.

4. Verfahren nach Anspruch 3, **dadurch gekennzeichnet, dass** das Lösungsmittel wenigstens 50 Vol.-% eines aprotischen polaren Lösungsmittels ausgewählt unter Carbonsäureamiden, Carbonsäureestern, Carbonaten, Nitrilen und Sulfoxiden umfasst.

5. Verfahren nach Anspruch 4, **dadurch gekennzeichnet, dass** das Lösungsmittel wenigstens 80 Gew.-% eines aprotischen polaren Lösungsmittels umfasst.

6. Verfahren nach einem der vorherigen Ansprüche, **dadurch gekennzeichnet, dass** man pro Mol der Verbindung II 0,9 bis 1,3 Mol des Enamins der Formel III einsetzt.

7. Verfahren nach einem der vorherigen Ansprüche, **dadurch gekennzeichnet, dass** man ein 3-Phenyl(thio)uracil oder ein 3-Phenyldithiouracil bereitstellt, worin R¹ gleich Wasserstoff ist, und diese Verbindung I anschließend
(A) mit einem Aminierungsmittel der Formel IV
H₂N-L¹ IV,
worin L¹ für eine nucleophil verdrängbare Abgangsgruppe steht,
umsetzt, wobei man eine Verbindung der Formel I erhält, worin
R¹ für Amino steht; und
die Variablen R², R³, X¹, X², X³, Ar und A die zuvor genannten Bedeutungen aufweisen; oder
(B) mit einem Alkylierungsmittel der Formel V 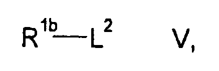
worin
R^{1b} C₁-C₆-Alkyl, C₁-C₆-Halogenalkyl, C₃-C₇-Cycloalkyl, C₂-C₆-Alkenyl, C₂-C₆-Halogenalkenyl, C₃-C₆-Alkinyl oder C₃-C₆-Halogenalkinyl ; und
L² eine nucleophil verdrängbare Abgangsgruppe;
bedeutet;
umsetzt, wobei man eine Verbindung der allgemeinen Formel I erhält, worin
R¹ die für R^{1b} genannten Bedeutungen hat; und
die Variablen R², R³, X¹, X², X³, Ar und A die zuvor genannten Bedeutungen aufweisen.

8. Verfahren nach einem der vorherigen Ansprüche, **dadurch gekennzeichnet, dass** das Phenyliso(thio)cyanat der Formel II durch die Formel IIA beschrieben wird 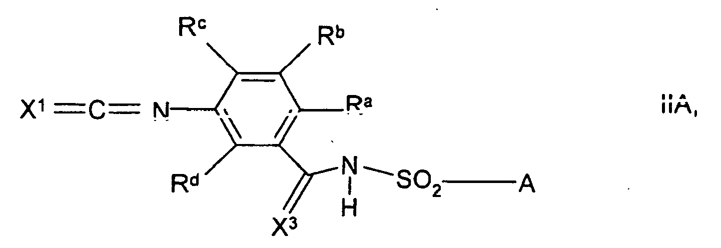 worin
X¹, X³ und A die zuvor genannte Bedeutung aufweisen und
R^{a}, R^{b}, R^{c} und R^{d} jeweils unabhängig voneinander
Wasserstoff, Halogen, Cyano, C₁-C₄-Alkyl oder C₁-C₄-Halogenalkyl bedeuten.

9. Verfahren nach Anspruch 8, **dadurch gekennzeichnet, dass** in Formel IIA
R^{a} Halogen, Cyano oder Trifluormethyl;
R^{c} Wasserstoff oder Halogen; und
R^{b} und R^{d} Wasserstoff
bedeuten.

10. Verfahren nach einem der vorherigen Ansprüche, **dadurch gekennzeichnet, dass** der Rest A für -NR⁵R⁶ steht, worin die Variablen R⁵ und R⁶ die folgenden Bedeutungen haben:
R⁵ und R⁶ unabhängig voneinander
Wasserstoff, C₁-C₁₀-Alkyl, C₂-C₁₀-Alkenyl oder C₂-C₁₀-Alkinyl, die unsubstituiert oder durch einen der folgenden Reste substituiert sein können:
C_{I}-C₄-Alkoxy, C₁-C₄-Alkylthio, CN, NO₂, Formyl, C₁-C₄-Alkylcarbonyl, C₁-C₄-Alkoxycarbonyl, C₁-C₄-Alkylaminocarbonyl, C₁-C₄-Dialkylaminocarbonyl, C₁-C₄-Alkylsulfinyl, C₁-C₄-Alkylsulfonyl, C₃-C₁₀-Cycloalkyl, 3- bis 8-gliedriges Heterocyclyl mit ein bis drei Heteroatomen ausgewählt unter O, S, N und einer Gruppe NR⁷
worin R⁷ für Wasserstoff, C₁-C₆-Alkyl, C₃-C₆-Alkenyl oder C₃-C₆-Alkinyl steht
Phenyl, das seinerseits 1, 2, 3 oder 4 Substituenten, ausgewählt unter Halogen, C₁-C₄-Alkyl, C₁-C₄-Alkoxy, C₁-C₄-Fluoralkyl, C₁-C₄-Alkyloxycarbonyl, Trifluormethylsulfonyl, C₁-C₃-Alkylamino, C₁-C₃-Dialkylamino, Formyl, Nitro oder Cyano, aufweisen kann;
C₁-C₁₀-Halogenalkyl, C₂-C₁₀-Halogenalkenyl, C₂-C₁₀-Halogenalkinyl, C₃-C₈-Cycloalkyl, C₃-C₁₀-Cycloalkenyl, 3- bis 8-gliedriges Heterocyclyl mit ein bis drei Heteroatomen, ausgewählt unter O, S, N und einer Gruppe NR⁷, worin R⁷ für Wasserstoff, C₁-C₆-Alkyl, C₃-C₆-Alkenyl oder C₃-C₆-Alkinyl steht,
Phenyl oder Naphthyl,
wobei C₃-C₈-Cycloalkyl, C₃-C₁₀-Cycloalkenyl, 3- bis 8-gliedriges Heterocyclyl, Phenyl oder Naphthyl ihrerseits durch 1, 2, 3 oder 4 Substituenten ausgewählt unter Halogen, C₁-C₄-Alkyl, C₁-C₄-Alkoxy, C₁-C₄-Fluoralkyl,
C₁-C₄-Alkyloxycarbonyl, Trifluormethylsulfonyl, Formyl, C₁-C₃-Alkylamino, C₁-C₃-Dialkylamino, Phenoxy, Nitro oder Cyano substituiert sein können, oder
R⁵ und R⁶ bilden gemeinsam einen gesättigten oder teilweise ungesättigten 5- bis 8-gliedrigen Stickstoffheterocyclus, der ein oder zwei Carbonylgruppen, Thiocarbonylgruppen und/oder ein oder zwei weitere Heteroatome, ausgewählt unter O, S, N und einer Gruppe NR₇,
worin R⁷ für Wasserstoff, C₁-C₆-Alkyl, C₃-C₆-Alkenyl oder C₃-C₆-Alkinyl steht,
als Ringglieder aufweisen kann; und der
durch C₁-C₄-Alkyl, C₁-C₄-Alkoxy und/oder C₁-C₄-Halogenalkyl substituiert sein kann.

11. Verfahren nach Anspruch 10, **dadurch gekennzeichnet, dass** R⁵ und R⁶ die folgenden Bedeutungen haben:
R⁵ und R⁶ unabhängig voneinander
Wasserstoff, C₁-C₆-Alkyl, das gegebenenfalls einen Substituenten ausgewählt aus der Gruppe Halogen, Cyano, C₁-C₄-Alkoxy, C₁-C₄-Alkoxycarbonyl, C₁-C₄-Alkylthio, C₃-C₈-Cycloalkyl, Furyl, Thienyl, 1,3-Dioxolanyl und Phenyl,
das seinerseits gegebenenfalls durch Halogen oder C₁-C₄-Alkoxy substituiert sein kann, tragen kann;
C₂-C₆-Alkenyl, C₂-C₆-Alkinyl, C₃-C₈-Cycloalkyl oder Phenyl, das gegebenenfalls 1
oder 2 Substituenten, ausgewählt aus der Gruppe Halogen, C₁-C₄-Alkyl, C₁-C₄-Fluoralkyl, C₁-C₄-Alkoxy, C₁-C₄-Alkoxycarbonyl, Nitro und C₁-C₃-Dialkylamino tragen kann;
Naphthyl oder Pyridyl; oder
R⁵ und R⁶ bilden zusammen einen fünf-, sechs- oder siebengliedrigen gesättigten oder ungesättigten Stickstoffheterocyclus, der ein weiteres Heteroatom ausgewählt unter N, O, und einer Gruppe NR⁷,
wobei R⁷ für Wasserstoff, C₁-C₆-Alkyl, C₃-C₆-Alkenyl oder C₃-C₆-Alkinyl steht;
als Ringglied enthalten kann,
und/oder durch ein, zwei oder drei Substituenten ausgewählt unter C₁-C₄-Alkyl und C₁-C₄-Halogenalkyl substituiert sein kann.

12. Verfahren nach einem der vorherigen Ansprüche, **dadurch gekennzeichnet, dass** X¹, X² und X³ jeweils für Sauerstoff stehen.

13. Verfahren nach einem der vorhergehenden Ansprüche, **dadurch gekennzeichnet, dass** R¹ für Wasserstoff, Amino oder C₁-C₄-Alkyl steht.

14. Verfahren nach einem der vorhergehenden Ansprüche, **dadurch gekennzeichnet, dass** R² für Wasserstoff, C₁-C₄-Alkyl oder C₁-C₄-Halogenalkyl steht.

15. Verfahren nach einem der vorhergehenden Ansprüche, **dadurch gekennzeichnet, dass** R³ für Wasserstoff steht.

16. Verfahren zur Herstellung von 3-Phenyl(thio)uracilen oder 3-Phenyldithiouracilen der Formel I, 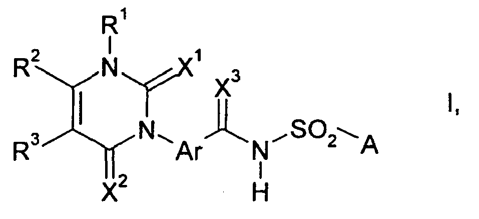 wobei
R¹ C₁-C₆-Akyl, C₁-C₆-Halogenalkyl, C₃-C₇-Cycloalkyl, C₂-C₆-Akenyl, C₂-C₆-Halogenalkinyl, C₃-C₆-Akinyl oder C₃-C₆-Halogenakinyl;
R² und R³ unabhängig voneinander Wasserstoff, C₁-C₆-Alkyl, C₁-C₆-Halogenalkyl, C₃-C₇-Cycloalkyl, C₂-C₆-Alkenyl; C₂-C₆-Halogenalkenyl, C₃-C₆-Alkinyl oder C₃-C₆-Halogenalkinyl;
X¹, X² und X³ unabhängig voneinander Sauerstoff oder Schwefel;
Ar Phenyl, das durch folgende Gruppen ein- oder mehrfach substituiert sein kann: Wasserstoff, Halogen, Cyano, C₁-C₄-Alkyl oder C₁-C₄-Halogenalkyl; und
A ein von einem primären oder sekundären Amin abgeleiteter Rest oder NH₂;
bedeutet,
**dadurch gekennzeichnet, dass** 3-Phenyl(thio)uracile oder 3-Phenyldithiouracile der Formel I, wobei R¹ für Wasserstoff steht, mit einem Alkylierungsmittel der Formel V 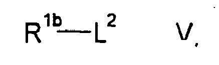 wobei L² für eine nucleophil verdrängbare Abgangsgruppe steht, und
R^{1b} C₁-C₆-Alkyl, C₁-C₆-Halogenalkyl, C₃-C₇-Cycloalkyl, C₂-C₆-Akenyl, C₂-C₆-Halogenalkenyl, C₃-C₆-Akinyl oder C₃-C₆-Halogenalkinyl bedeutet,
umgesetzt werden.

## Claims

1. A process for preparing 3-phenyl(thio)uracils or 3-phenyldithiouracils of the formula I 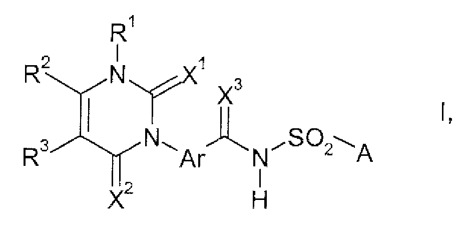 where the variables are each defined as follows:
R¹ is hydrogen, cyano, amino, C₁-C₆-alkyl, C₁-C₃-cyanoalkyl, C₁-C₆-haloalkyl, C₁-C₆-haloalkoxy, C₃-C₇-cycloalkyl, C₂-C₆-alkenyl, C₂-C₆-haloalkenyl, C₃-C₆-alkynyl, C₃-C₆-haloalkynyl or phenyl-C₁-C₄-alkyl;
R² and R³ are each independently hydrogen, C₁-C₆-alkyl, C₁-C₆-haloalkyl, C₃-C₇-cycloalkyl, C₂-C₆-alkenyl, C₂-C₆-haloalkenyl, C₃-C₆-alkynyl or C₃-C₆-haloalkynyl;
X¹, X² and X³ are each independently oxygen or sulfur;
Ar is phenyl, which may be mono- or polysubstituted by the following groups: hydrogen, halogen, cyano, C₁-C₄-alkyl or C₁-C₄-haloalkyl; and
A is a radical derived from a primary or secondary amine or NH₂;
comprising the reaction of a phenyl iso(thio)cyanate of the formula II 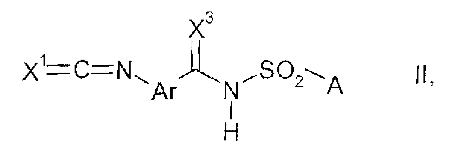 where the variables X¹, X³, Ar and A are each as defined above,
with an enamine of the formula III 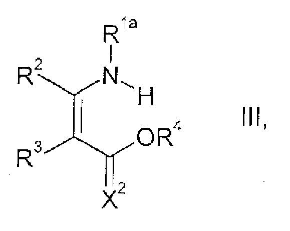 where
R^{1a} is as defined above for R¹ with the exception of amino;
R², R³ and X² are each as defined above; and
R⁴ is C₁-C₆-alkyl, C₁-C₆-haloalkyl, C₁-C₃-alkoxy-C₁-C₃-alkyl, C₁-C₃-alkylthio-C₁-C₃-alkyl, C₂-C₆-alkenyl, C₂-C₆-haloalkenyl, C₃-C₆-alkynyl, C₃-C₆-haloalkynyl, C₃-C₇-cycloalkyl, C₁-C₆-cyanoalkyl or benzyl which is itself unsubstituted or substituted on the phenyl ring by methyl, methoxy, methylthio, halogen, nitro or cyano;
in the presence of from 1.8 to 2.6 base equivalents per mole of the phenyl iso(thio)cyanate of the formula II;
and, if appropriate, in a further step, the reaction of the resulting 3-phenyl(thio)uracil or 3-phenyldithiouracil of the formula I where R¹=R^{1a}, where R¹ is hydrogen, with an aminating agent of the formula IV
H₂N-L¹ IV,
where L¹ is a nucleophilically displaceable leaving group
to give 3-phenyl(thio)uracils or 3-phenyldithiouracils of the formula I where R¹ = amino.

2. The process according to claim 1, wherein the reaction is effected in the presence of a base which is selected from alkali metal and alkaline earth metal carbonates, alkali metal and alkaline earth metal alkoxides, alkali metal and alkaline earth metal hydrides and tertiary amines.

3. The process according to either of the preceding claims, wherein the reaction is effected in at least one aprotic polar solvent, and the aprotic polar solvent has a water content of from 0 to 0.5% by weight, based on the total amount of compound II, compound III and solvent.

4. The process according to claim 3, wherein the solvent comprises at least 50% by volume of an aprotic polar solvent selected from carboxamides, carboxylic esters, carbonates, nitriles and sulfoxides.

5. The process according to claim 4, wherein the solvent comprises at least 80% by weight of an aprotic polar solvent.

6. The process according to any of the preceding claims, wherein from 0.9 to 1.3 mol of the enamine of the formula III are used per mole of the compound II.

7. The process according to any of the preceding claims, wherein a 3-phenyl(thio)-uracil or a 3-phenyldithiouracil, where R¹ is hydrogen, is prepared and this compound I is subsequently
(A) reacted with an aminating agent of the formula IV
H₂N-L¹ IV
where L¹ is a nucleophilically displaceable leaving group to obtain a compound of the formula I where
R¹ is amino; and
the variables R², R³, X¹, X², X³, Ar and A are each as defined above; or
(B) reacted with an alkylating agent of the formula V
R^{1b}-L² V
where
R^{1b} is C₁-C₆-alkyl, C₁-C₆-haloalkyl, C₃-C₇-cycloalkyl, C₂-C₆-alkenyl, C₂-C₆-haloalkenyl, C₃-C₆-alkynyl or C₃-C₆-haloalkynyl; and
L² is a nucleophilically displaceable leaving group;
to obtain a compound of the general formula I where
R¹ is as defined for R^{1b}; and
the variables R², R³, X¹, X², X³, Ar and A are each as defined above.

8. The process according to any of the preceding claims, wherein the phenyl iso(thio)cyanate of the formula II is described by the formula IIA 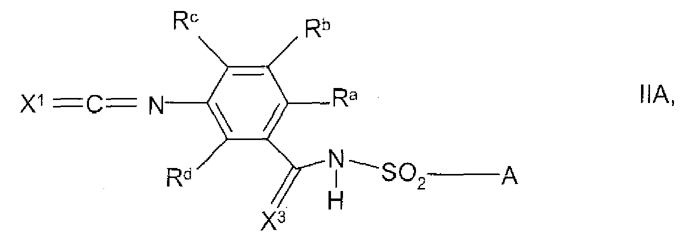 where
X¹, X³ and A are each as defined above and
R^{a}, R^{b}, R^{c} and R^{d} are each independently
hydrogen, halogen, cyano, C₁-C₄-alkyl or C₁-C₄-haloalkyl.

9. The process according to claim 8, wherein, in formula IIA,
R^{a} is halogen, cyano or trifluoromethyl;
R^{c} is hydrogen or halogen; and
R^{b} and R^{d} are each hydrogen.

10. The process according to any of the preceding claims, wherein the A radical is -NR⁵R⁶ where the variables R⁵ and R⁶ are each defined as follows:
R⁵ and R⁶ are each independently
hydrogen, C₁-C₁₀-alkyl, C₂-C₁₀₋-alkenyl or C₂-C₁₀-alkynyl, each of which may be unsubstituted or substituted by one of the following radicals:
C₁-C₄-alkoxy, C₁-C₄-alkylthio, CN, NO₂, formyl, C₁-C₄-alkylcarbonyl, C₁-C₄-alkoxycarbonyl, C₁-C₄-alkylaminocarbonyl, C₁-C₄-dialkylaminocarbonyl, C₁-C₄-alkylsulfinyl, C₁-C₄-alkylsulfonyl, C₃-C₁₀-cycloalkyl, 3- to 8-membered heterocyclyl having from one to three heteroatoms selected from O, S, N and an NR⁷ group
where R⁷ is hydrogen, C₁-C₆-alkyl, C₃-C₆-alkenyl or C₃-C₆-alkynyl, phenyl which may itself have 1, 2, 3 or 4 substituents selected from halogen, C₁-C₄-alkyl, C₁-C₄-alkoxy, C₁-C₄-fluoroalkyl, C₁-C₄-alkyloxycarbonyl, trifluoromethylsulfonyl, C₁-C₃-alkylamino, C₁-C₃-dialkylamino, formyl, nitro or cyano;
C₁-C₁₀-haloalkyl, C₂-C₁₀-haloalkenyl, C₂-C₁₀-haloalkynyl, C₃-C₈-cycloalkyl, C₃-C₁₀-cycloalkenyl, 3- to 8-membered heterocyclyl having from one to three heteroatoms selected from O, S, N and an NR⁷ group where R⁷ is hydrogen, C₁-C₆-alkyl, C₁-C₆-alkenyl or C₃-C₆-alkynyl,
phenyl or naphthyl,
where C₃-C₈-cycloalkyl, C₃-C₁₀-cycloalkenyl, 3- to 8-membered heterocyclyl, phenyl or naphthyl, may themselves each be substituted by 1, 2, 3 or 4 substituents selected from halogen, C₁-C₄-alkyl, C₁-C₄-alkoxy, C₁-C₄-fluoroalkyl,
C₁-C₄-alkyloxycarbonyl, trifluoromethylsulfonyl, formyl, C₁-C₃-alkylamino, C₁-C₃-dialkylamino, phenoxy, nitro or cyano; or
R⁵ and R⁶ together form a saturated or partially unsaturated 5- to 8-membered nitrogen heterocycle which may have, as ring members, one or two carbonyl groups, thiocarbonyl groups and/or one or two further heteroatoms selected from O, S, N and an NR⁷ group
where R⁷ is hydrogen, C₁-C₆-alkyl, C₃-C₆-alkenyl or C₃-C₆-alkynyl,
and which may be substituted
by C₁-C₄-alkyl, C₁-C₄-alkoxy and/or C₁-C₄-haloalkyl.

11. The process according to claim 10, wherein R⁵ and R⁶ are each defined as follows:
R⁵ and R⁶ are each independently
hydrogen, C₁-C₆-alkyl which may if appropriate carry a substituent selected from the group consisting of halogen, cyano, C₁-C₄-alkoxy, C₁-C₄-alkoxycarbonyl, C₁-C₄-alkylthio, C₃-C₈-cycloalkyl, furyl, thienyl, 1,3-dioxolanyl and phenyl
which may itself optionally be substituted by halogen or C₁-C₄-alkoxy; C₂-C₆-alkenyl, C₂-C₆-alkynyl, C₃-C₈-cycloalkyl or phenyl
which may if appropriate carry 1 or 2 substituents selected from the group consisting of halogen, C₁-C₄-alkyl, C₁-C₄-fluoroalkyl, C₁-C₄-alkoxy, C₁-C₄-alkoxycarbonyl, nitro and C₁-C₃-dialkylamino;
naphthyl or pyridyl; or
R⁵ and R⁶ together form a five-, six- or seven-membered saturated or
unsaturated nitrogen heterocycle which may contain, as a ring member, one further heteroatom selected from N, O and an NR⁷ group
where R⁷ is hydrogen, C₁-C₆-alkyl, C₃-C₆-alkenyl or C₃-C₆-alkynyl,
and/or may be substituted by one, two or three substituents selected from C₁-C₄-alkyl and C₁-C₄-haloalkyl.

12. The process according to any of the preceding claims, wherein X¹, X² and X³ are each oxygen.

13. The process according to any of the preceding claims, wherein R¹ is hydrogen, amino or C₁-C₄-alkyl.

14. The process according to any of the preceding claims, wherein R² is hydrogen, C₁-C₄-alkyl or C₁-C₄-haloalkyl.

15. The process according to any of the preceding claims, wherein R³ is hydrogen.

16. A process for preparing 3-phenyl(thio)uracils or 3-phenyldithiouracils of the formula I 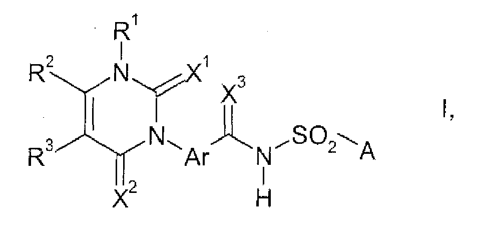 where
R¹ is C₁-C₆-alkyl, C₁-C₆-haloalkyl, C₃-C₇-cycloalkyl, C₂-C₆-alkenyl, C₂-C₆-haloalkenyl, C₃-C₆-alkynyl or C₃-C₆-haloalkynyl;
R² and R³ are each independently
hydrogen, C₁-C₆-alkyl, C₁-C₆-haloalkyl, C₃-C₇-cycloalkyl, C₂-C₆-alkenyl, C₂-C₆-haloalkenyl, C₃-C₆-alkynyl or C₃-C₆-haloalkynyl;
X¹, X² and X³ are each independently oxygen or sulfur;
Ar is phenyl, which may be mono- or polysubstituted by the following groups: hydrogen, halogen, cyano, C₁-C₄-alkyl or C₁-C₄-haloalkyl; and
A is a radical derived from a primary or secondary amine or NH₂,
wherein 3-phenyl(thio)uracils or 3-phenyldithiouracils of the formula I, where R¹ is hydrogen, are reacted with an alkylating agent of the formula V 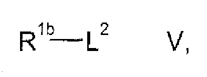 where L² is a nucleophilically displaceable leaving group, and
R^{1b} is C₁-C₆-alkyl, C₁-C₆-haloalkyl, C₃-C₇-cycloalkyl, C₂-C₆-alkenyl, C₂-C₆-haloalkenyl, C₃-C₆-alkynyl or C₃-C₆-haloalkynyl.

## Revendications

1. Procédé de préparation de 3-phényl(thio)uraciles ou de 3-phényldithiouraciles de formule I 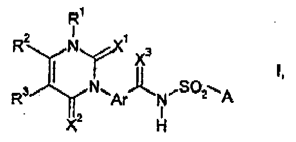 dans laquelle les variables ont les significations suivantes :
R¹ hydrogène, cyano, amino, alkyle en C₁-C₆, cyanoalkyle en C₁-C₃, halogénoalkyle en C₁-C₆, halogénoalcoxy en C₁-C₆, cycloalkyle en C₃-C₇, alcényle en C₂-C₆, halogénoalcényle en C₂-C₆, alcynyle en C₃-C₆, halogénoalcynyle en C₃-C₆ ou phénylalkyle en C₁-C₄,
R² et R³ indépendamment l'un de l'autre, hydrogène, alkyle en C₁-C₆, halogénoalkyle en C₁-C₆, cycloalkyle en C₃-C₇, alcényle en C₂-C₆, halogénoalcényle en C₂-C₆, alcynyle en C₃-C₆ ou halogénoalcynyle en C₃-C₆,
X¹, X² et X³ indépendamment les uns des autres, oxygène ou soufre,
Ar phényle, qui peut être substitué une ou plusieurs fois par les groupements suivants : hydrogène, halogène, cyano, alkyle en C₁-C₄ ou halogénoalkyle en C₁-C₄, et
A un radical dérivé d'une amine primaire ou secondaire ou NH₂,
comprenant la réaction d'un phényliso(thio)cyanate de formule II 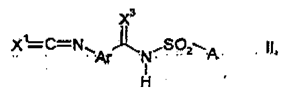 dans laquelle les variables X¹, X³, Ar et A présentent les significations indiquées ci-dessus,
avec une énamine de formule III 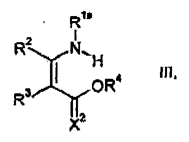 dans laquelle
R^{1a} présente les significations indiquées ci-dessus pour R¹, l'exception d'amino,
R², R³ et X² présentent les significations indiquées ci-dessus,
R⁴ représente un radical alkyle en C₁-C₆, halogénoalkyle en C₁-C₆, (C₁-C₃)alcoxy(C₁-C₃)alkyle, (C₁-C₃)alkylthio(C₁-C₃)alkyle, alcényle en C₂-C₆, halogénoalcényle en C₂-C₆, alcynyle en C₃-C₆, halogénoalcynyle en C₃-C₆, cycloalkyle en C₃-C₇, cyanoalkyle en C₁-C₆ ou benzyle, qui est pour sa part non substitué ou substitué par méthyle, méthoxy, méthylthio, halogène, nitro ou cyano sur le noyau phényle,
en présence de 1, à à 2, 6 équivalents de base par mole du phényliso(thio)cyanate de formule II ;
le cas échéant, dans une autre étape, la réaction du 3-phényl(thio)uracile ou du 3-phényldithiouracile obtenu de formule I avec R¹ = R^{1a}, lorsque R¹ représente de l'hydrogène, avec un agent d'amination de formule IV
H₂N-L¹ (IV)
dans laquelle L¹ représente un groupe partant nucléophile déplaçable,
pour former des 3-phényl(thio)uraciles ou des 3-phényldithiouraciles de formule I, avec R¹ = amino.

2. Procédé suivant la revendication 1, **caractérisé en ce que** la réaction est réalisée en présence d'une base, qui est choisie parmi les carbonates alcalins et alcalino-terreux, les alcoolates de métaux alcalins et alcalino-terreux, les hydrures alcalins et alcalino-terreux et les amines tertiaires.

3. Procédé suivant l'une quelconque des revendications qui précèdent, **caractérisé en ce que** la réaction est réalisée dans au moins un solvant aprotique polaire, et **en ce que** le solvant aprotique polaire présente une teneur en eau allant de 0 à 0,5% en poids, par rapport à la quantité totale de composé II, de composé III et de solvant.

4. Procédé suivant la revendication 3, **caractérisé en ce que** le solvant comprend au moins 50% en volume d'un solvant aprotique polaire choisi parmi des amides d'acides carboxyliques, des esters d'acides carboxylique, des carbonates, des nitriles et des sulfoxydes.

5. Procédé suivant la revendication 4, **caractérisé en ce que** le solvant comprend au moins 80% en volume d'un solvant aprotique polaire.

6. Procédé suivant l'une quelconque des revendications qui précèdent, **caractérisé en ce que** l'on met en oeuvre par mole de composé II, 0,9 à 1,3 mole de l'énamine de formule III.

7. Procédé suivant l'une quelconque des revendications qui précèdent, **caractérisé en ce que** l'on prépare un 3-phényl(thio)uracile ou un 3-phényldithiouracile, où R¹ est de l'hydrogène, et qu'ensuite, ce composé I est mis à réagir :
A) avec un agent d'amination de formule IV
H₂N-L¹ (IV)
dans laquelle L¹ représente un groupe partant nucléophile déplaçable,
où l'on obtient un composé de formule I, dans laquelle
R¹ représente un radical amino,
les variables R², R³, X¹, X², X³, Ar et A présentent les significations indiquées ci-dessus, ou
B) avec un agent d'alkylation de formule V
R^{1b}-L² (V)
dans laquelle
R^{1b} représente un radical alkyle en C₁-C₆, halogénoalkyle en C₁-C₆, cycloalkyle en C₃-C₇, alcényle en C₂-C₆, halogénoalcényle en C₂-C₆, alcynyle en C₃-C₆ ou halogénoalcynyle en C₃-C₆, et
L² représente un groupe partant nucléophile déplaçable,
où l'on obtient un composé de formule générale I, dans laquelle R¹ a les significations données pour R^{1b}, et
les variables R², R³, X¹, X², X³, Ar et A présentent les significations indiquées ci-dessus.

8. Procédé suivant l'une quelconque des revendications qui précèdent, **caractérisé en ce que** le phényliso(thio)cyanate de formule II est décrit par la formule IIA 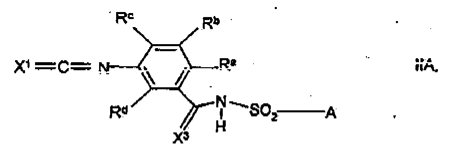 dans laquelle :
X¹, X³ et A présentent les significations indiquées ci-dessus, et
R^{a}, R^{b}, R^{c} et R^{d} représentent à chaque fois indépendamment les uns des autres, de l'hydrogène, halogène, cyano, alkyle en C₁-C₄ ou halogénoalkyle en C₁-C₄.

9. Procédé suivant la revendication 8, **caractérisé en ce que**, dans la formule IIA,
R^{a} représente un halogène, un cyano ou un trifluorométhyle,
R^{c} représente de l'hydrogène ou un halogène, et
R^{b} et R^{d} représentent de l'hydrogène.

10. Procédé suivant l'une quelconque des revendications qui précèdent, **caractérisé en ce que** le radical A représente -NR⁵R⁶, où les variables R⁵ et R⁶ présentent les significations suivantes :
R⁵ et R⁶ représentent indépendamment l'un de l'autre de l'hydrogène, ou un radical alkyle en C₁-C₁₀, alcényle en C₂-C₁₀ ou alcynyle en C₂-C₁₀, qui peuvent être non substitués ou substitués par un des radicaux suivants :
alcoxy en C₁-C₄, alkylthio en C₂-C₄, CN, NO₂, formyle, (C₁-C₄)alkylcarbonyle, (C₁-C₄)alcoxycarbonyle, (C₁-C₄)alkylaminocarbonyle, di(C₁-C₄)alkylaminocarbonyle, (C₁-C₄)alkylsulfinyle, (C₁-C₄)alkylsulfonyle, cycloalkyle en C₃-C₁₀, un hétérocyclyle ayant 3 à 8 membres, avec un à trois hétéroatomes choisis parmi O, S, N et un groupe NR⁷,
où R⁷ représente de l'hydrogène ou un radical alkyle en C₁-C₆, alcényle en C₃-C₆ ou alcynyle en C₃-C₆,
phényle, qui peut présenter pour sa part 1, 2, 3 ou 4 substituants, choisis parmi un halogène ou un radical alkyle en C₁-C₄, alcoxy en C₁-C₄, fluoroalkyle en C₁-C₄, (C₁-C₄)alkyloxycarbonyle, trifluorométhylsulfonyle, (C₁-C₃)alkylamino, di(C₁-C₃)alkylamino, formyle, nitro ou cyano,
halogénoalkyle en C₁-C₁₀, halogénoalcényle en C₂-C₁₀, halogénoalcynyle en C₂-C₁₀, cycloalkyle en C₃-C₈, cycloalcényle en C₃-C₁₀, hétérocyclyle ayant 3 à 8 membres, avec un à trois hétéroatomes choisis parmi O, S, N et un groupement NR⁷, où R⁷ représente de l'hydrogène ou un radical alkyle en C₁-C₆, alcényle en C₃-C₆ ou alcynyle en C₃-C₆,
phényle ou naphtyle,
où des radicaux cycloalkyle en C₃-C₈, cycloalcényle en C₃-C₁₀, hétérocyclyle ayant 3 à 8 membres, phényle ou naphtyle peuvent être substitués pour leur part par 1, 2, 3 ou 4 substituants, choisis parmi un halogène ou un radical alkyle en C₁-C₄, alcoxy en C₁-C₄, fluoroalkyle en C₁-C₄, (C₁-C₄)alkyloxy carbonyle, trifluorométhylsulfonyle, formyle, (C₁-C₃)alkylamino, di(C₁-C₃)alkylamino, phénoxy, nitro ou cyano, ou
R⁵ et R⁶ peuvent former ensemble un hétérocycle azoté saturé ou partiellement insaturé, ayant 5 à 8 membres, qui peut présenter comme membres du cycle, un ou deux groupes carbonyle, groupes thiocarbonyle et/ou un ou deux autres hétéroatomes, choisis parmi O, S, N et un groupement NR⁷,
où R⁷ représente de l'hydrogène ou un radical alkyle en C₁-C₆, alcényle en C₃-C₆ ou alcynyle en C₃-C₆, et
qui peut être substitué par des radicaux alkyle en C₁-C₄, alcoxy en C₁-C₄ et/ou halogénoalkyle en C₁-C₄.

11. Procédé suivant la revendication 10, **caractérisé en ce que** R⁵ et R⁶ ont les significations suivantes :
R⁵ et R⁶ représentent indépendamment l'un de l'autre de l'hydrogène ou des radicaux alkyle en C₁-C₆, qui peut porter, le cas échéant, un substituant choisi dans le groupe formé par halogène, cyano, alcoxy en C₁-C₄, (C₁-C₄)alcoxycarbonyle, alkylthio en C₁-C₄, cycloalkyle en C₃-C₈, furyle, thiényle, 1,3-dioxolanyle et phényle, qui peut pour sa part être le cas échéant substitué par un halogène, ou un alcoxy en C₁-C₄,
alcényle en C₂-C₆, alcynyle en C₂-C₆, cycloalkyle en C₃-C₈, ou phényle, qui peut le cas échéant porter 1 ou 2 substituants choisis dans le groupe formé par halogène, alkyle en C₁-C₄, fluoroalkyle en C₁-C₄, alcoxy en C₁-C₄, (C₁-C₄)alcoxycarbonyle, nitro et di(C₁-C₃)alkylamino,
naphtyle ou pyridyle, ou
R⁵ et R⁶ forment ensemble un hétérocycle azoté saturé ou insaturé, ayant cinq, six ou sept membres, qui peut présenter comme membre du cycle un autre hétéroatome choisi parmi N, O et un groupement NR⁷,
où R⁷ représente de l'hydrogène, un radical alkyle
en C₁-C₆, alcényle en C₃-C₆ ou alcynyle en C₃-C₆,
et/ou qui peut être substitué par un, deux ou trois substituants choisis parmi des radicaux alkyle en C₁-C₄ et halogénoalkyle en C₁-C₄.

12. Procédé suivant l'une quelconque des revendications qui précèdent, **caractérisé en ce que** X¹, X² et X³ représentent à chaque fois de l'oxygène.

13. Procédé suivant l'une quelconque des revendications qui précèdent, **caractérisé en ce que** R¹ représente de l'hydrogène ou un radical amino ou alkyle en C₁-C₄.

14. Procédé suivant l'une quelconque des revendications qui précèdent, **caractérisé en ce que** R² représente de l'hydrogène ou un radical alkyle en C₁-C₄ ou halogénoalkyle en C₁-C₄.

15. Procédé suivant l'une quelconque des revendications qui précèdent, **caractérisé en ce que** R³ représente de l'hydrogène.

16. Procédé de préparation de 3-phényl(thio)uraciles ou de 3-phényldithiouraciles de formule I 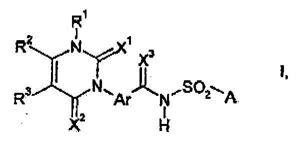 dans laquelle
R¹ représente un radical alkyle en C₁-C₆, halogénoalkyle en C₁-C₆, cycloalkyle en C₃-C₇, alcényle en C₂-C₆, halogénoalcényle en C₂-C₆, alcynyle en C₃-C₆ ou halogénoalcynyle en C₃-C₆ ;
R² et R³ représentent indépendamment l'un de l'autre de l'hydrogène ou un radical alkyle en C₁-C₆, halogénoalkyle en C₁-C₆, cycloalkyle en C₃-C₇, alcényle en C₂-C₆, halogénoalcényle en C₂-C₆, alcynyle en C₃-C₆ ou halogénoalcynyle en C₃-C₆,
X¹, X² et X³ représentent indépendamment les uns des autres de l'oxygène ou du soufre,
Ar représente un phényle, qui peut être substitué une ou plusieurs fois par les groupements suivants : hydrogène, halogène, cyano, alkyle en C₁-C₄ ou halogénoalkyle en C₁-C₄, et
A représente un radical dérivé d'une amine primaire ou secondaire ou NH₂ ,
**caractérisé en ce que** les 3-phényl(thio)uraciles ou les 3-phényldithiouraciles de formule I, dans laquelle R¹ représente de l'hydrogène, sont mis à réagir avec un agent d'alkylation de formule V
R^{1b}-L² (V)
dans laquelle L² représente un groupe partant nucléophile déplaçable, et
R^{1b} représente un radical alkyle en C₁-C₆, halogénoalkyle en C₁-C₆, cycloalkyle en C₃-C₇, alcényle en C₂-C₆, halogénoalcényle en C₂-C₆, alcynyle en C₃-C₆ ou halogénoalcynyle en C₃-C₆.
